# EUROPEAN PATENT APPLICATION

(11) **EP 3 536 343 A1**
(43) Date of publication of application: **11.09.2019**
(21) Application number: 17868078.1
(22) Date of filing: 01.11.2017
(51) Int. Cl.: A61K 47/08, A61K 9/127, A61K 31/7105, A61K 45/00, A61K 47/18, A61K 47/24, A61K 47/28, A61P 17/00, C12N 15/113

(54) **SKIN FIBROSIS TREATMENT AGENT**

(30) Priority: 02.11.2016 JP 2016215686
(71) Applicant: Nitto Denko Corporation, Ibaraki-shi, Osaka 567-8680 (JP)
(72) Inventor: TESHIMA, Takanori, Sapporo-shi Hokkaido 060-0808 (JP); HASHIMOTO, Daigo, Sapporo-shi Hokkaido 060-0808 (JP); NIITSU, Yoshiro, Sapporo-shi Hokkaido 064-0823 (JP); MINOMI, Kenjiro, Ibaraki-shi Osaka 567-8680 (JP)
(74) Representative: Hoffmann Eitle
(86) International application number: PCT/JP2017/039478
(87) International publication number: WO 2018/084168

(57) **Abstract**

The present invention relates to: a carrier for delivering a retinoid-containing substance used by extracellular-matrix-producing cells in the skin; a skin fibrosis treatment agent using said carrier; a preparation kit for said treatment agent; a production method for said carrier; and a production method for said treatment agent.

## Description

### [Technical Field]

The present disclosure relates to a carrier for promoting the delivery of a substance to extracellular matrix-producing cells in the skin, a composition for treating skin fibrosis utilizing the carrier, a treatment method for skin fibrosis, and the like.

### [Background Art]

In systemic sclerosis or chronic graft-versus-host disease (cGVHD) occurring after allogeneic hematopoietic stem cell transplantation, severe skin fibrogenesis often occurs (Non-Patent Document 1 and Non-Patent Document 2). Skin fibrogenesis not only causes an aesthetic problem, but also causes, for example, joint range of motion restriction, trismus, mask-like facies, respiratory disorder, or the like due to sclerema when it becomes severe to significantly deteriorate the quality of life (QOL) of patients. At present, as a treatment method therefor, administration of an immunosuppressive agent is performed, however, it cannot be necessarily said that the effect thereof is sufficient, and also it is accompanied by widespread immunosuppression, and therefore, occurrence of infectious diseases or recurrence of tumors may be caused. In order to safely and effectively treat such patients, development of a fibrogenesis inhibitor without immunosuppressive effects has been awaited.

### [Citation List]

Non-Patent Document 1: Sticherling M. J Dtsch Dermatol Ges. 2012, 10(11): 783-91
Non-Patent Document 2: Martires KJ et al. Blood 2011, 118(15): 4250-7

### [Disclosure of Invention]

### Problems to be solved by the Invention

Some embodiments of the present disclosure have been made to solve the problems as described above, and an object of the present disclosure is to provide a novel skin fibrosis treatment agent and a novel treatment method for skin fibrosis.

### Means for Solving the Problems

Some embodiments of the present disclosure relate to the following.
<1> A carrier for delivering a substance to extracellular matrix-producing cells in the skin, containing a retinoid as a component for promoting the delivery of a substance to extracellular matrix-producing cells in the skin.
<2> The carrier according to the above <1>, wherein the retinoid is contained as a retinoid conjugate.
<3> The carrier according to the above <1> or <2>, further containing a lipid.
<4> A pharmaceutical composition for treating skin fibrosis, containing the carrier according to any one of the above <1> to <3>, and a drug that controls the activity or growth of extracellular matrix-producing cells in the skin.
<5> A pharmaceutical composition for regenerating a normal skin tissue from a fibrotic skin tissue, containing the carrier according to any one of the above <1> to <3>, and a drug that controls the activity or growth of extracellular matrix-producing cells in the skin.
<6> The pharmaceutical composition according to the above <4> or <5>, wherein the drug that controls the activity or growth of extracellular matrix-producing cells in the skin is selected from the group consisting of a substance that inhibits the production or secretion of an extracellular matrix component, a cell growth inhibitor, an apoptosis inducer, and a TIMP inhibitor.
<7> The pharmaceutical composition according to the above <6>, wherein the substance that inhibits the production or secretion of an extracellular matrix component is an HSP47 inhibitor.
<8> The pharmaceutical composition according to any one of the above <4> to <7>, which is in a form to be prepared before use.
<9> A kit for preparing the pharmaceutical composition according to any one of the above <4> to <8>, including one or more containers containing a drug that controls the activity or growth of extracellular matrix-producing cells in the skin, a retinoid and/or a retinoid conjugate, and a carrier constituent substance other than the retinoid as needed singly or in combination.
<10> A method for producing a carrier for delivering a substance to extracellular matrix-producing cells in the skin, including a step of blending a retinoid and/or a retinoid conjugate as a component for promoting the delivery of a substance to extracellular matrix-producing cells in the skin.
<11> A method for producing a pharmaceutical composition for treating skin fibrosis or a pharmaceutical composition for regenerating a normal skin tissue from a fibrotic skin tissue, including a step of blending a retinoid as a component for promoting the delivery of a substance to extracellular matrix-producing cells in the skin and a drug that controls the activity or growth of extracellular matrix-producing cells in the skin as an active ingredient.

### Advantageous Effects of Invention

By using the carrier of the present disclosure, an active ingredient can be efficiently delivered to a site of action (for example, extracellular matrix-producing cells in the skin), and therefore, the cure, suppression of progression, and prevention of onset of skin fibrosis, for which there has been no fundamental treatment method to date, are made possible, and this carrier thus contributes greatly to human medical care and veterinary medical care.

Further, the carrier of the present disclosure can be combined with any medicinal agent (for example, an existing therapeutic drug for skin fibrosis) to enhance its efficiency of action, and therefore, there is also an advantage that the range of pharmaceutical applications is wide, and the production of an effective treatment agent can be simply performed.

### [Brief Description of Drawings]

FIG. 1 is a graph showing inhibition of expression of HSP47 by HSP47 siRNA-containing retinoid-conjugated liposomes in skin myofibroblasts.
FIG. 2 is a diagram showing a regimen for dosing to bleomycin (BLM) skin fibrogenesis-induced model mice.
FIG. 3 shows photographic diagrams of MT stained skin sections collected from skin fibrosis model mice.
FIG. 4 shows (A) graphs showing thickening of the dermis collected from skin fibrosis model mice and (B) the results of measurement of collagen dot density capable of evaluating collagen deposition on an image basis.
FIG. 5 shows (A and B) graphs showing the expression levels of mRNA of Col1a1 and HSP47, respectively, by the qPCR method, and (C) the results of a hydroxyproline assay.
FIG. 6 is a diagram showing a regimen for producing skin fibrogenesis model mice by cGVHD.
FIG. 7 shows (A) tissue images of MT stained skin sections and graphs showing thickening of the dermis collected from cGVHD model mice and (B) the results of measurement of collagen dot density.
FIG. 8 shows photographic diagrams showing the results of immunofluorescent staining of skin sections collected from cGVHD model mice.
FIG. 9 is a diagram showing a regimen for dosing to cGVHD model mice.
FIG. 10 shows graphs showing (A) the percent survival and (B) the body weight change in a treated group with HSP47 siRNA-containing retinoid-conjugated liposomes and a control group. Diarrhea or death due to cGVHD was observed in both groups at the same level, and therefore, a difference in percent survival or body weight change was not observed. An increase in adverse event in the treated group was not observed.
FIG. 11 shows (A) tissue images of MT stained skin sections collected from the treated group with HSP47 siRNA-containing retinoid-conjugated liposomes and a control group of cGVHD model mice, (B) a graph showing thickening of the dermis, and (C) a graph showing the results of measurement of collagen dot density.
FIG. 12 is a graph showing the results of expression level of mRNA of HSP47 by the qPCR method.
FIG. 13 is a graph showing the results of a collagen assay.
FIG. 14 shows photographic diagrams showing localization of HSP47 and α-SMA-positive cells in skin tissues of a cGVHD model mouse (Allo) and a control mouse (Syn). The scale bars indicate 50 µm.
FIG. 15 shows graphs showing (A) the collagen amount reduction effect and (B) the dermal thickening reduction effect of HSP47 siRNA-containing retinoid-conjugated liposomes in cGVHD model mice.
FIG. 16 shows photographic diagrams showing suppression of the expression of HSP47 by HSP47 siRNA-containing retinoid-conjugated liposomes in cGVHD model mice. The lower images are enlarged images of the upper images.
FIG. 17 shows graphs showing the effect of administering HSP47 siRNA-containing retinoid-conjugated liposomes on (A) the number of both CD4 and CD8-positive cells contained in the thymus of cGVHD model mice, and (B) the number of CD4-positive cells or (C) the number of CD8-positive cells contained in the spleen. N.S indicates that a significant difference was not observed (Mann-Whitney U test, P>0.5).
FIG. 18 shows graphs showing the effect of administering HSP47 siRNA-containing retinoid-conjugated liposomes on the percentage of donor T cells (left) and CS11b-posituve donor myeloid cells (right).
FIG. 19 shows photographic diagrams showing the delivery of a label by retinoid-conjugated liposomes in a normal skin tissue (left) or a fibrotic lesion (right) of bleomycin-induced skin fibrogenesis model mice. The lower image is an enlarged image of the upper image. The scale bars indicate 50 µm.
FIG. 20 is a graph showing the effect of administering HSP47 siRNA-containing retinoid-conjugated liposomes on the dermal thickening of a normal skin tissue or a fibrotic lesion of bleomycin-induced skin fibrogenesis model mice.
FIG. 21 shows graphs showing the dermal thickening reduction effect (left) and the collagen amount reduction effect (right) of HSP47 siRNA-containing retinoid-conjugated liposomes on skin fibrogenesis established by cGVHD.

### [Description of Embodiments]

One aspect of the present disclosure relates to a carrier for delivering a substance to extracellular matrix-producing cells in the skin, containing a retinoid as a component for promoting the delivery of a substance to extracellular matrix-producing cells (herein sometimes referred to as "target cells") in the skin. In one embodiment, the carrier of the present disclosure contains the retinoid in an effective amount for promoting the delivery of a substance to extracellular matrix-producing cells in the skin. Further, one embodiment of the carrier of the present disclosure relates to a carrier with which the delivery of a substance to extracellular matrix-producing cells in the skin is promoted by a retinoid.

In the present disclosure, the extracellular matrix-producing cells in the skin are cells that are present in the skin and have an ability to produce an extracellular matrix, and include, for example, fibroblasts, pericytes, fibrocytes, and myofibroblasts that are present in the skin. The extracellular matrix-producing cells that are present in the skin can include not only those derived from cells that are present in the skin, but also those derived from fibrocytes in circulating blood. Further, the extracellular matrix-producing cells in the skin may be extracellular matrix-producing cells (e.g. fibroblasts or myofibroblasts) transformed from endothelial cells (e.g. vascular endothelial cells, etc.) by endothelial-mesenchymal transformation (EndoMT) or transformed from epithelial cells (e.g. keratinocytes, etc.) by epithelial-mesenchymal transformation (EMT) .

The extracellular matrix-producing cells in the skin may be present at one or two or more sites selected from the group consisting of epidermis, dermis, and a subcutaneous tissue. In one embodiment, the extracellular matrix-producing cells in the skin are present in the dermis and/or a subcutaneous tissue. In one embodiment, the extracellular matrix-producing cells in the skin are present in a fibrotic lesion of the skin. The fibrotic lesion of the skin can occur at one or two or more sites selected from the group consisting of epidermis, dermis, and a subcutaneous tissue. In a specific embodiment, the extracellular matrix-producing cells in the skin are present in a fibrotic lesion in the dermis and/or a subcutaneous tissue.

In one embodiment, the extracellular matrix-producing cells in the skin express CRABP (cellular retinoic acid binding protein). The CRABP includes CRABP1 and CRABP2. Therefore, the extracellular matrix-producing cells in the above-mentioned embodiment express CRABP1 and/or CRABP2. In one embodiment, the extracellular matrix-producing cells in the skin express α-SMA (alpha-smooth muscle actin). In one embodiment, the extracellular matrix-producing cells in the skin express vimentin. In one embodiment, the extracellular matrix-producing cells in the skin express HSP47 (heat shock protein 47). In a specific embodiment, the extracellular matrix-producing cells in the skin express CRABP and HSP47.

The expression of a marker such as CRABP, α-SMA, vimentin, or HSP47 in cells can be confirmed by, for example, detecting the expression of each marker in the cells. The gene sequences of CRABP1, CRABP2, α-SMA, vimentin, and HSP47 are known, and antibodies against these have also been developed, and therefore, the expression thereof can be detected by a known nucleic acid or protein detection method, for example, but without being limited to, an immunoprecipitation method utilizing an antibody, EIA (enzyme immunoassay) (e.g. ELISA (emzyme-linked immunosorbent assay), etc.), RIA (radio immuno assay) (e.g. IRMA (immunoradiometric assay), RAST (radioallergosorbent test), RIST (radioimmunosorbent test), etc.), a Western blotting method, an immunohistochemical method, an immunocytochemical method, a flow cytometry method, various hybridization methods utilizing a nucleic acid that specifically hybridizes with a nucleic acid encoding CRABP1, CRABP2, α-SMA, vimentin, or HSP47, or a unique fragment thereof or a transcription product (e.g. mRNA) or a splicing product of the nucleic acid, a Northern blotting method, a Southern blotting method, various PCR methods, or the like.

In the present disclosure, the "retinoid" refers to natural and synthetic retinoids including first generation, second generation, and third generation retinoids. Examples of a naturally occurring retinoid include, but are not limited to, 11-cis-retinal, all-trans retinol, retinyl palmitate, all-trans retinoic acid, and 13-cis-retinoic acid. Further, the term "retinoid" encompasses retinoic acid, retinol, retinal, and derivatives and analogues thereof. In one embodiment, the retinoid has a skeleton in which four isoprenoid units are linked in a head-to-tail manner.

Non-limiting examples of the retinoid include, for example, retinol (including all-trans retinol), retinal, retinoic acid (including tretinoin), an ester of retinol and a fatty acid, an ester of an aliphatic alcohol and retinoic acid, etretinate, isotretinoin, adapalene, acitretine, tazarotene, retinol palmitate, and retinoid derivatives such as saturated derivatives of retinol, retinoic acid, retinal, etretinate, isotretinoin, acitretine, and the like, and vitamin A analogues such as fenretinide (4-HPR) and bexarotene.

In one embodiment, the retinoid includes retinoic acid and analogues thereof. In one embodiment, the retinoic acid analogue shows binding affinity to CRABP. Non-limiting examples of the retinoic acid analogue showing binding affinity to CRABP include, for example, SRI 2965-38, Ro 13-7410, Ro31-3689, St 80, SRI 6409-40, TTNN, TTAB, Ch 80, Az 80, Am 580, Am 555, Am 80, 5,6-epoxy-retinoic acid, 4-OH-retinoic acid, 4-oxo-retinoic acid, retinoic acid glucuronide, and 9-cis-retinoic acid (see Trown et al., Cancer Res. 1980 Feb; 40(2): 212-20, Sani et al., Cancer Res. 1984; 44(1): 190-5, Lotan et al., Cancer Res. 1980; 40(4): 1097-102, Keidel et al., Eur J Biochem. 1993; 212(1): 13-26, Jetten et al., Cancer Res. 1987; 47 (13) : 3523-7, etc.). The binding affinity of a compound to CRABP can be determined by, for example, a competitive binding assay using radioactively labeled retinoic acid or the like.

The retinoid may be present as a retinoid conjugate in which a retinoid and another compound are bonded to each other. Such another compound may be a retinoid of the same type as the retinoid or a different retinoid from the retinoid, or a non-retinoid compound (e.g. a lipid or the like) . The retinoid and another compound may be directly bonded to each other through a condensation reaction or the like or may be bonded to each other through a linker.

In one embodiment, the retinoid conjugate is a compound having a structure I.

X-Y-Z I

In the formula,
X is a retinoid or a lipid,
Z is a retinoid, and
Y is a linker.

The retinoid or the lipid contained as X and Z in the structure I may be a free retinoid or a part of a lipid according to the bonding style to the linker, or may be one in which a linking group is added to a free retinoid or a lipid so as to provide an attachment point to the linker. For example, when retinoic acid and a linker having an amino group at an end are bonded by dehydration condensation, the OH group contained in the carboxyl group at position 15 of the retinoic acid and H contained in the amino group of the linker are detached, and therefore, X and Z become a portion in which the OH group is excluded from the retinoic acid.

X and Z may each independently contain one or more retinoids. X and Z may each independently contain 1, 2, 3, 4, or 5 retinoids. When X and/or Z contains more than one retinoids, the respective retinoids may be the same as or different from one another.

Examples of the lipid contained as X in the structure I include, but are not limited to, a lipid containing one or more alkyl chains, and a head group containing an amide. In a specific embodiment, the lipid contained as X in the structure I is selected from the group consisting of DODC, HEDODC, DSPE, DOPE, and DC-6-14.

In the present disclosure, the "linker" includes any linkers that can bond the retinoid. The linker may have a straight-chain shape or a branched shape. In one embodiment, the linker is a chemical bond. The chemical bond includes a single bond, a double bond, and a triple bond. In another embodiment, the linker includes polyethylene glycol (PEG). The average molecular weight of the PEG is not particularly limited, and may be, for example, within a range of 200 to 5000, 500 to 3000, 550 to 2000, or the like. The PEG may contain one or more amide groups and/or amino groups. The PEG may contain one or more linking groups such as lysine residues. In one embodiment, the PEG contains one or more amide groups and one or more lysine residues. In another embodiment, the linker is selected from the group consisting of Glu, hexanoyl, and Gly₃ or GluNH.

In some embodiments, the PEG may be bis-amide-PEG, tris-amide-PEG, tetra-amide-PEG, Lys-bis-amide-PEG-Lys, Lys-tris-amide-PEG-Lys, Lys-tetra-amide-PEG-Lys, or Lys-PEG-Lys. In some embodiments, the PEG has repeating units: -CH₂CH₂O- whose number is 5 to 50, 6 to 40, 7 to 30, 8 to 25, 9 to 20, 10 to 15, etc.

A non-limiting example of the compound in which X and Z are each two retinoic acids and the linker is Lys-bis-amide-PEG-Lys is shown below.

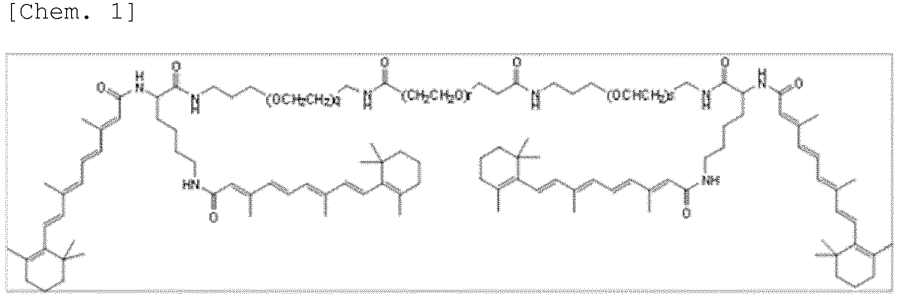

In the formula, q, r, and s are each independently 1, 2, 3, 4, 5, 6, 7, 8, 9, or 10.

Non-limiting examples of the compound having the structure I are shown below.

### [Table 1]

**Table 1: Non-limiting examples of compound having structure I (X is retinoid)**

| Compound No. | Structure/chemical name |
|---|---|
| XR1 | 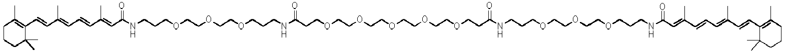 |
| | N1,N19-bis((16E, 18E,20E,22E)-17,21-dimethyl-15-oxo-23-(2,6,6-trimethylcyclohex-1-en-1-yl)-4,7,10-trioxa-14-azatricosa-16,18,20,22-tetraen-1-yl)-4,7,10,13,16-pentaoxan onadecane-1,19-diamide |
| XR2 | 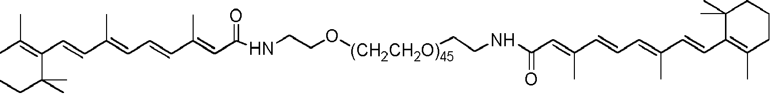 |
| | (2E,2'E,4E,4'E,6E,6'E,8E,8'E)-N,N'-(3,6,9,12,15,18,21,24,27,30,33,36,39,42,45,48,51, 54,57,60,63,66,69,72,75,78,81,84,87,90,93,96,99,102,105,108,111,114,117,120,123,1 26,129,132,135,138-hexatetracontaoxatetracontahectane-1,140-diyl)bis(3,7-dimethyl-9-(2,6,6-trimethylcyclohex-1-en-1-yl)nona-2,4,6,8-tetraenamide) |
| XR3 | 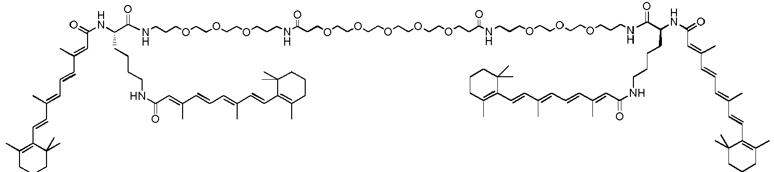 |
| | N1,N19-bis((S,23E,25E,27E,29E)-16-((2E,4E,6E,8E)-3,7-dimethyl-9-(2,6,6-trimethylcy clo-hex-1-en-1-yl)nona-2,4,6,8-tetraenamido)-24,28-dimethyl-15,22-dioxo-30-(2,6,6-tri methylcyclohex-1-en-1-yl)-4,7,10-trioxa-14,21-diazatriaconta-23,25,27,29-tetraen-1-yl) -4,7,10,13,16-pentaoxanonadecane-1,19-diamide |
| XR4 | 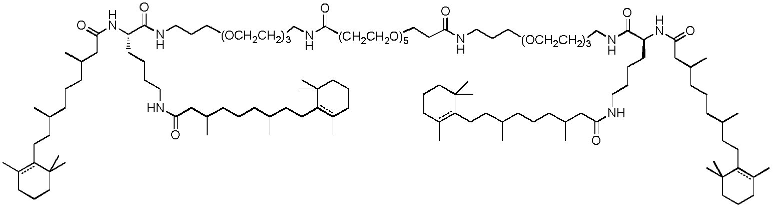 |
| | N1,N19-bis((16S)-16-(3,7-dimethyl-9-(2,6,6-trimethylcyclohex-1-en-1-yl)nonanamido)-24,28-dimethyl-15,22-dioxo-30-(2,6,6-trimethylcyclohex-1-en-1-yl)-4,7,10-trioxa-14,21 -diazatriacontyl)-4,7,10,13,16-pentaoxanonadecane-1,19-diamide |

**[Table 2]**

| | |
|---|---|
| XR5 | 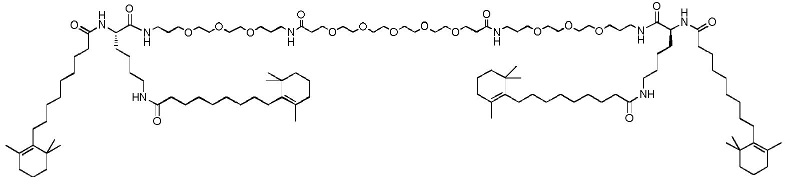 |
| | N1,N19-bis((S)-15,22-dioxo-30-(2,6,6-trimethylcyclohex-1-en-1-yl)-16-(9-(2,6,6-trimethyl cyclohex-1-en-1-yl)nonanamido)-4,7,10-trioxa-14,21-diazatriacontyl)-4,7,10,13,16-penta oxanonadecane-1,19-diamide |
| XR6 | 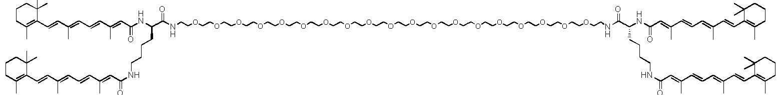 |
| | (2E,2'E,2"E,4E,4'E,4"E,6E,6'E,6"E,8E,8'E,8"E)-N,N',N"-((5R,69R,76E,78E,80E,82E)-77,8 1-dimethyl-6,68,75-trioxo-83-(2,6,6-trimethylcyclohex-1-en-1-yl)-10,13,16,19,22,25,28,31 ,34,37,40,43,46,49,52,55,58,61,64-nonadecaoxa-7,67,74-triazatrioctaconta-76,78,80,82-tetraene-1,5,69-triyl)tris(3,7-dimethyl-9-(2,6,6-trimethylcyclohex-1-en-1-yl)nona-2,4,6,8-t etraenamide) |
| XR7 | 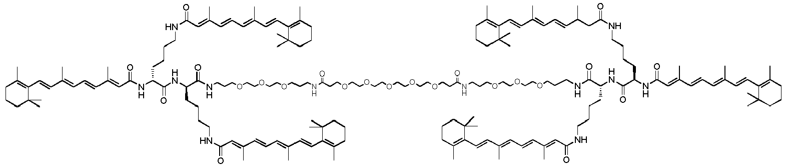 |
| XR8 | 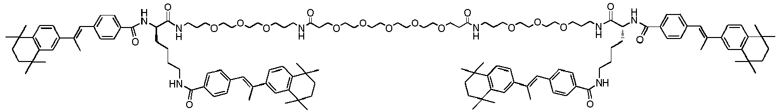 |
| | N1,N19-bis((R)-1,8-dioxo-7-(4-((E)-2-(5,5,8,8-tetramethyl-5,6,7,8-tetrahydro-naphthalen-2-yl)prop-1-en-1-yl)benzamido)-1-(4-((E)-2-(5,5,8,8-tetramethyl-5,6,7,8-tetrahydronaphth alen-2-yl)prop-1-en-1-yl)phenyl)-13,16,19-trioxa-2,9-diazadocosan-22-yl)-4,7,10,13,16-p entaoxanonadecane-1,19-diamide |
| XR9 | 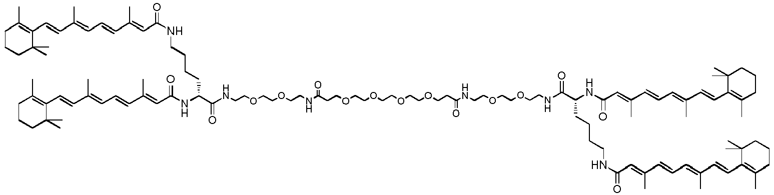 |
| | N1,N19-bis((R,18E,20E,22E,24E)-11-((2E,4E,6E,8E)-3,7-dimethyl-9-(2,6,6-trimethyl-cycl ohex-1-en-1-yl)nona-2,4,6,8-tetraenamido)-19,23-dimethyl-10,17-dioxo-25-(2,6,6-trimeth ylcyclohex-1-en-1-yl)-3,6-dioxa-9,16-diazapentacosa-18,20,22,24-tetraen-1-yl)-4,7,10,13 -tetraoxahexadecane-1,16-diamide |

### [Table 3]

**Table 2: Non-limiting examples of compound having structure I (X is lipid)**

| | |
|---|---|
| XL1 | 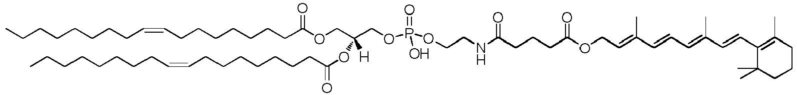 |
| | (Z)-(2R)-3-(((2-(5-(((2E,4E,6E,8E)-3,7-dimethyl-9-(2,6,6-trimethylcyclohex-1-en-1-yl)non a-2,4,6,8-tetraen-1-yl)oxy)-5-oxopentanamido)ethoxy)(hydroxy)phosphoryl)oxy)propane-1,2-diyl dioleate |
| XL2 | 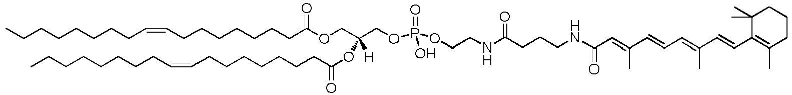 |
| | (Z)-(2R)-3-(((2-(4-((2E,4E,6E,8E)-3,7-dimethyl-9-(2,6,6-trimethylcyclohex-1-en-1-yl)nona -2,4,6,8-tetraenamido)butanamido)ethoxy)(hydroxy)phosphoryl)oxy)propane-1,2-diyl dioleate |
| XL3 | 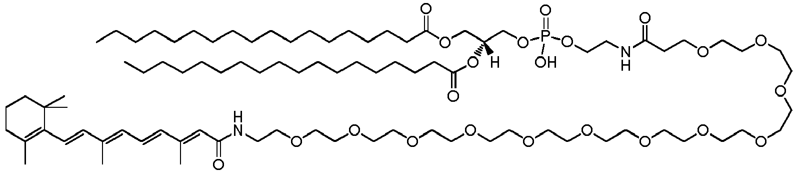 |
| | (2R)-3-(((((45E,47E,49E,51E)-46,50-dimethyl-4,44-dioxo-52-(2,6,6-trimethylcyclohex-1-e n-1-yl)-7,10,13,16,19,22,25,28,31,34,37,40-dodecaoxa-3,43-diazadopentaconta-45,47,4 9,51-tetraen-1-yl)oxy)(hydroxy)phosphoryl)oxy)propane-1,2-diyl distearate |
| XL4 | 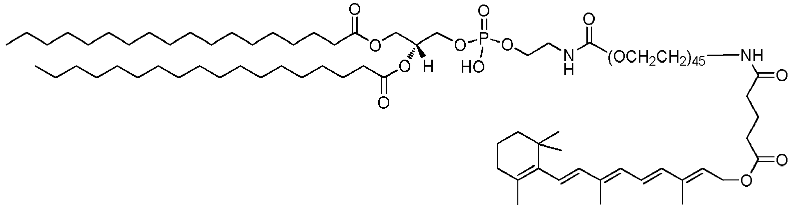 |
| XL5 | 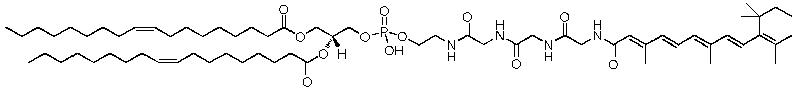 |
| | (Z)-(2R)-3-(((((14E,16E,18E,20E)-15,19-dimethyl-4,7,10,13-tetraoxo-21-(2,6,6-trimethylc yclohex-1-en-1-yl)-3,6,9,12-tetraazahenicosa-14,16,18,20-tetraen-1-yl)oxy)(hydroxy)pho sphoryl)oxy)propane-1,2-diyl dioleate |

**[Table 4]**

| | |
|---|---|
| XL6 | 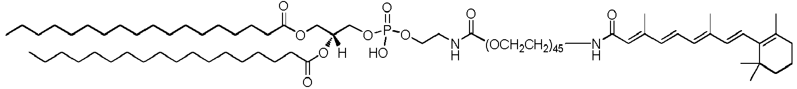 |
| XL7 | 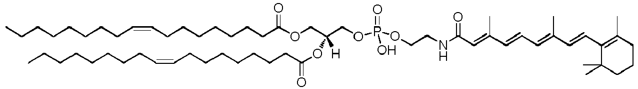 |
| | (Z)-(2R)-3-(((2-((2E,4E,6E,8E)-3,7-dimethyl-9-(2,6,6-trimethylcyclohex-1-en-1-yl)nona-2, 4,6,8-tetraenamido)ethoxy)(hydroxy)phosphoryl)oxy)propane-1,2-diyl dioleate |
| XL8 | 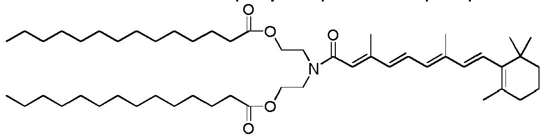 |
| | (((2E,4E,6E,8E)-3,7-dimethyl-9-(2,6,6-trimethylcyclohex-1-en-1-yl)nona-2,4,6,8-tetraeno yl)azanediyl)bis(ethane-2,1-diyl)ditetradecanoate |
| XL9 | 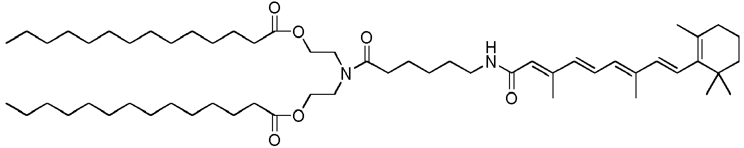 |
| | ((6-((2E,4E,6E,8E)-3,7-dimethyl-9-(2,6,6-trimethylcyclohex-1-en-1-yl)nona-2,4,6,8-tetrae namido)hexanoyl)azanediyl)bis(ethane-2,1-diyl)ditetradecanoate |

When the retinoid or the retinoid conjugate includes a stereoisomer, the compound includes respective stereoisomers thereof or any mixture of the stereoisomers. The retinoid or the retinoid conjugate is also sometimes substituted with one or two or more substituents. The retinoid or the retinoid conjugate not only includes one in an isolated state, but also includes a retinoid or a retinoid conjugate in a state of being dissolved or mixed in a medium capable of dissolving or retaining this.

The carrier of the present disclosure may be constituted by such a retinoid and/or a retinoid conjugate by itself, or may be constituted by bonding or incorporating the retinoid and/or the retinoid conjugate to or into a carrier constituent component other than this. Therefore, the carrier of the present disclosure may contain a carrier constituent component other than the retinoid or the retinoid conjugate. Such a component is not particularly limited and any material known in the medical and pharmaceutical field can be used, but is preferably a material that can incorporate the retinoid and/or the retinoid conjugate therein or can bond thereto.

In one embodiment, the carrier constituent component other than the retinoid and the retinoid conjugate includes a lipid. Non-limiting examples of such a lipid include phospholipids such as glycerophospholipids, sphingolipids such as sphingomyelin, sterols such as cholesterol, vegetable oils such as soybean oil and poppy oil, mineral oils, and lecithins such as egg yolk lecithin. More specific examples of the lipid include dimyristoylphosphatidylcholine (DMPC), dipalmitoylphosphatidylcholine (DPPC), distearoylphosphatidylcholine (DSPC), dioleylphosphatidylethanolamine (DOPE), dilauroylphosphatidylcholine (DLPC), sterols such as cholesterol, N-(α-trimethylammonioacetyl)-didodecyl-D-glutamate chloride (TMAG), N,N',N'',N'''-tetramethyl-N,N',N'',N'''-tetrapalmitylspermi ne (TMTPS), 2,3-dioleyloxy-N-[2(sperminecarboxamido)ethyl]-N,N-dimethyl -1-propanaminium trifluoroacetate (DOSPA), N-[1-(2,3-dioleyloxy)propyl]-N,N,N-trimethylammonium chloride (DOTMA), dioctadecyldimethylammonium chloride (DODAC), didodecylammonium bromide (DDAB), 1,2-dioleyloxy-3-trimethylammoniopropane (DOTAP), 3β-[N-(N',N'-dimethylaminoethane)carbamoyl]cholesterol (DC-Chol), 1,2-dimyristoyloxypropyl-3-dimethylhydroxyethyl ammonium bromide (DMRIE), and O,O'-ditetradecanoyl-N-(α-trimethylammonioacetyl)diethanol amine chloride (DC-6-14).

The lipid may be a cationic lipid or a non-cationic lipid, for example, an anionic lipid or a neutral lipid. In one embodiment, the carrier contains a cationic lipid. The cationic lipid is particularly useful for introducing a nucleic acid molecule or the like having a negative charge into cells. In another embodiment, the carrier contains an ionizable cationic lipid. The ionizable cationic lipid has such a property that it is non-ionic when the pH is not lower than PKa, but becomes cationic when the pH is lower than PKa. In some embodiments, the ionizable cationic lipid has a tertiary amine. In some embodiments, the PKa of the ionizable cationic lipid may be 7.0 or more, 7.5 or more, 7.6 or more, 7.8 or more, 8.0 or more, or the like. In order to distinguish from the ionizable cationic lipid, a cationic lipid that is always positively charged is sometimes referred to as "always-charged cationic lipid".

Other non-limiting examples of the lipid include always-charged cationic lipids and PEG-conjugated lipids (PEG-lipids) described in WO 2012/170952 and ionizable cationic lipids described in WO 2013/185116.

The always-charged cationic lipid is a compound represented by the formula I: 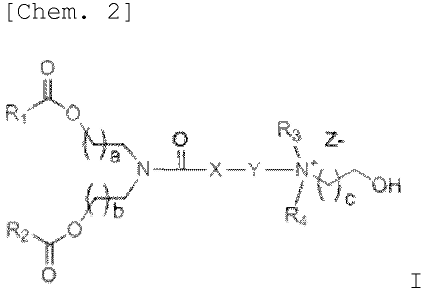 wherein R₁ and R₂ are independently selected from the group consisting of C₁₀ to C₁₈ alkyl, C₁₂ to C₁₈ alkenyl, and an oleyl group, R₃ and R₄ are independently selected from the group consisting of C₁ to C₆ alkyl and C₂ to C₆ alkanol, X is selected from the group consisting of -CH₂-, -S-, and -O- or absent, Y is selected from -(CH₂)ₙ, -S(CH₂)ₙ, -O(CH₂)ₙ-, thiophene, -SO₂(CH₂)ₙ-, and an ester, n=1 to 4, a=1 to 4, b=1 to 4, c=1 to 4, and Z is a counterion.

Non-limiting examples of the always-charged cationic lipid include, for example, the following lipids.

### [Table 5]

**Table 3: Non-limiting examples of always-charged cationic lipid**

| Lipid No. | Structure/chemical name |
|---|---|
| P1 | 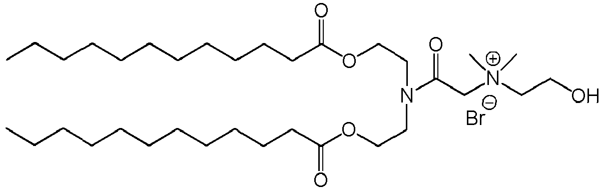 |
| | 2-(bis(2-(dodecanoyloxy)ethyl)amino)-N-(2-hydroxyethyl)-N,N-dimethyl-2-oxoethanaminiu m bromide |
| P2 | 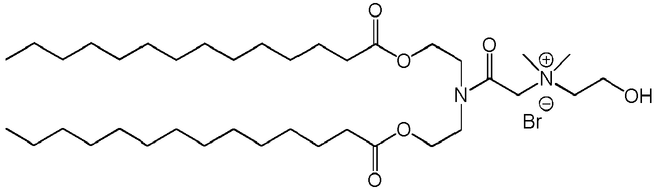 |
| | 2-(bis(2-(tetradecanoyloxy)ethyl)amino)-N-(2-hydroxyethyl)-N,N-dimethyl-2-oxoethan-amin ium bromide |
| P3 | 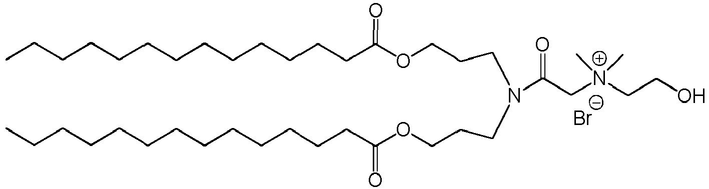 |
| | 2-(bis(3-(tetradecanoyloxy)propyl)amino)-N-(2-hydroxyethyl)-N,N-dimethyl-2-oxo-ethanami nium bromide |
| P4 | 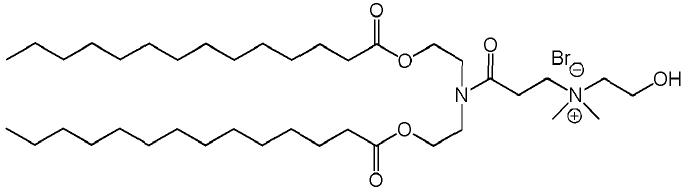 |
| | 3-(bis(2-(tetradecanoyloxy)ethyl)amino)-N-(2-hydroxyethyl)-N,N-dimethyl-3-oxopropan-1-a minium bromide |

**[Table 6]**

| | |
|---|---|
| P5 | 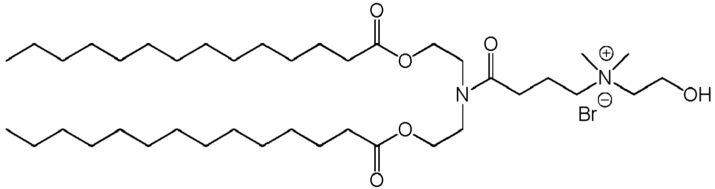 |
| | 4-(bis(2-(tetradecanoyloxy)ethyl)amino)-N-(2-hydroxyethyl)-N,N-dimethyl-4-oxobutan-1-a minium bromide |
| P6 | 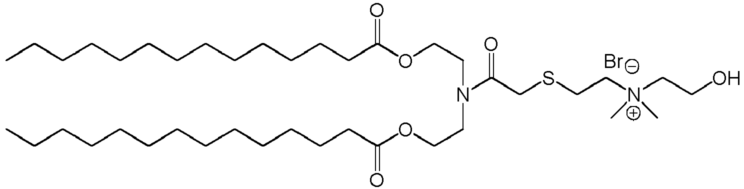 |
| | 2-((2-(bis(2-(tetradecanoyloxy)ethyl)amino)-2-oxoethyl)thio)-N-(2-hydroxyethyl)-N,N-dimet hylethanaminium bromide |
| P7 | 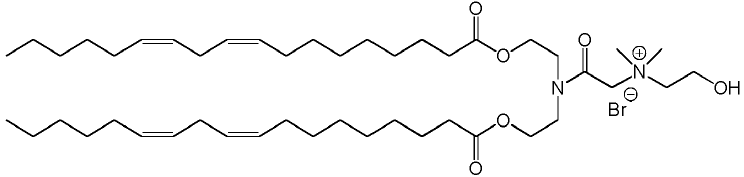 |
| P8 | 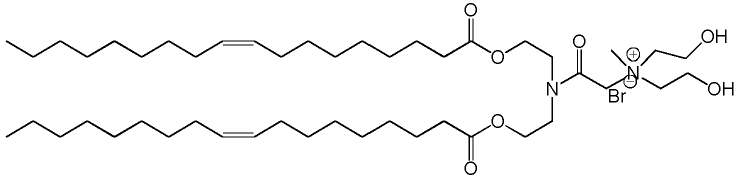 |
| P9 | 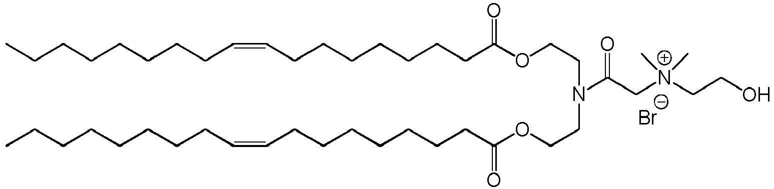 |

**[Table 7]**

| | |
|---|---|
| P10 | 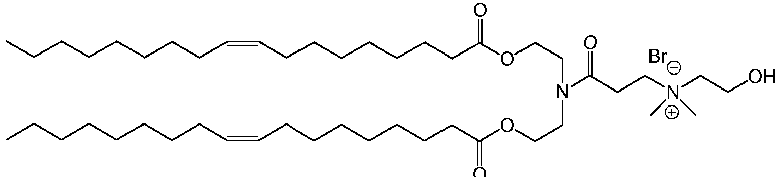 |
| | 3-(bis(2-(oleoyloxy)ethyl)amino)-N-(2-hydroxyethyl)-N,N-dimethyl-3-oxopropan-1-aminium bromide |
| P11 | 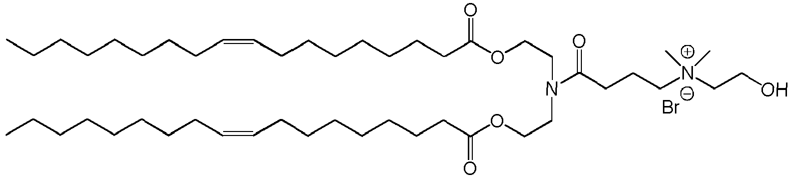 |
| | 4-(bis(2-(oleoyloxy)ethyl)amino)-N-(2-hydroxyethyl)-N,N-dimethyl-4-oxobutan-1-aminium bromide |
| P12 | 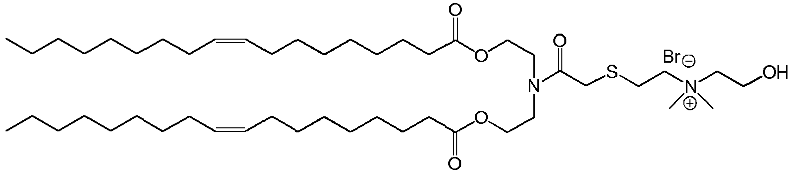 |
| P13 | 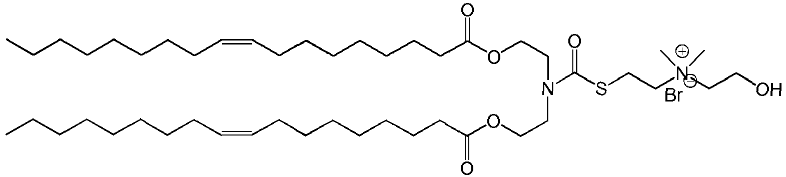 |
| | 2-((bis(2-(oleoyloxy)ethyl)carbamoyl)thio)-N-(2-hydroxyethyl)-N,N-dimethylethan-aminium bromide |
| P14 | 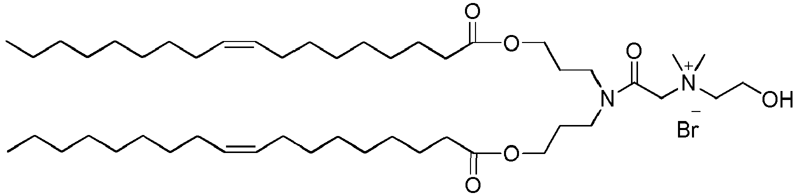 |
| | 2-(bis(3-(oleoyloxy)propyl)amino)-N-(2-hydroxyethyl)-N,N-dimethyl-2-oxoethanaminium bromide |

**[Table 8]**

| | |
|---|---|
| P15 | 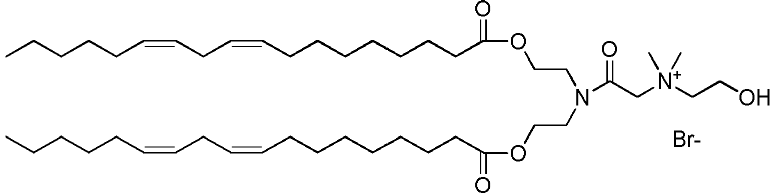 |
| | 2-(bis(2-((9Z,12Z)-octadeca-9,12-dienoyloxy)ethyl)amino)-N-(2-hydroxyethyl)-N,N-dimethy 1-2-oxoethanaminium bromide |

Non-limiting examples of the PEG-lipid include, for example, 1,2-dimyristoyl-sn-glycero-3-phosphoethanolamine-N-PEG (PEG-DMPE), 1,2-dipalmitoyl-sn-glycero-3-phosphoethanolamine-N-PEG (PEG-DPPE), 1,2-distearoyl-sn-glycero-3-phosphoethanolamine-N-PEG (PEG-DSPE), 1,2-dioleoyl-sn-glycero-3-phosphoethanolamine -N-PEG (PEG-DOPE), and PEG-ceramide. The molecular weight of the PEG is not particularly limited, and may be, for example, from about 200 to about 5000, from about 500 to about 3000, and from about 550 to about 2000, and more specifically about 550, about 750, about 1000, about 1250, about 2000, or the like. Non-limiting examples of the PEG include, for example, PEG 550, PEG 750, PEG 1000, PEG 1250, and PEG 2000.

The ionizable cationic lipid is a compound represented by the formula II: 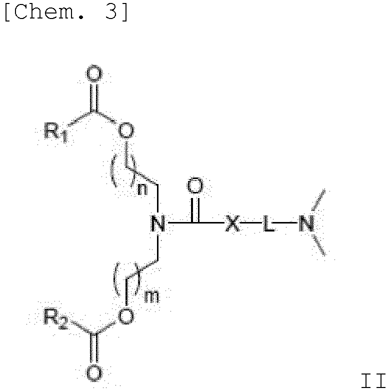 wherein, n and m are independently 1, 2, 3, or 4, R₁ and R₂ are independently C₁₀ to C₁₈ alkyl or C₁₂ to C₁₈ alkenyl, X is -CH₂-, S, O, N, or absent, L is C₁₋₄ alkylene, -S-C₁₋₄ alkylene, -O-C₁₋₄ alkylene, -O-C (O) -C₁₋₄ alkylene, -S(O)₂-C₁₋₄ alkylene, or 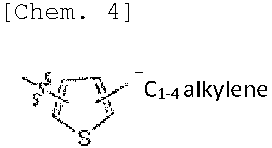 or is joined to 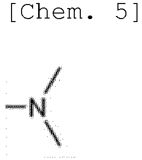 to form 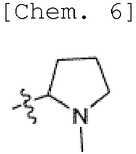 or a pharmaceutically acceptable salt thereof.

Non-limiting examples of the ionizable cationic lipid include, for example, the following lipids.

### [Table 9]

**Table 4: Non-limiting examples of ionizable cationic lipid**

| Lipid No. | Structure/chemical name |
|---|---|
| I1 | 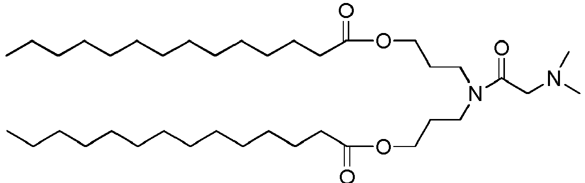 |
| | ((2-(dimethylamino)acetyl)azanediyl)bis(propane-3,1-diyl)ditetradecanoate |
| I2 | 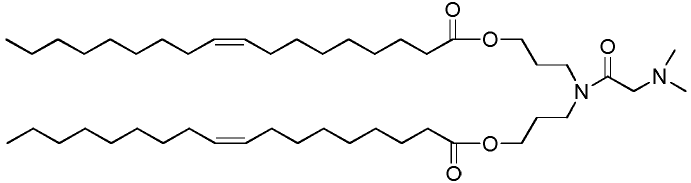 |
| | (Z)-((2-(dimethylamino)acetyl)azanediyl)bis(propane-3,1-diyl)dioleate |
| I3 | 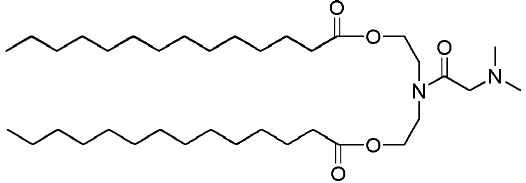 |
| | ((2-(dimethylamino)acetyl)azanediyl)bis(ethane-2,1-diyl)ditetradecanoate |
| I4 | 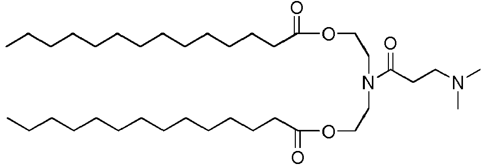 |
| | ((3-(dimethylamino)propanoyl)azanediyl)bis(ethane-2,1-diyl)ditetradecanoate |
| I5 | 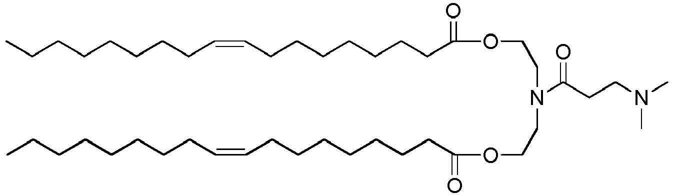 |
| | (Z)-((3-(dimethylamino)propanoyl)azanediyl)bis(ethane-2,1-diyl)dioleate |

**[Table 10]**

| | |
|---|---|
| I6 | 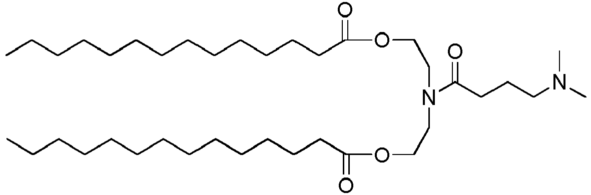 |
| | ((4-(dimethylamino)butanoyl)azanediyl)bis(ethane-2,1-diyl)ditetradecanoate |
| I7 | 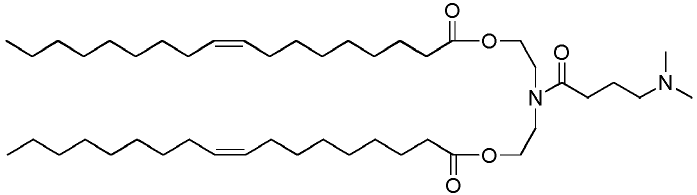 |
| | (Z)-((4-(dimethylamino)butanoyl)azanediyl)bis(ethane-2,1-diyl)dioleate |
| I8 | 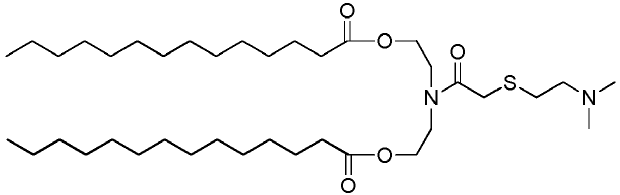 |
| | ((2-((2-(dimethylamino)ethyl)thio)acetyl)azanediyl)bis(ethane-2,1-diyl)ditetradecanoate |
| I9 | 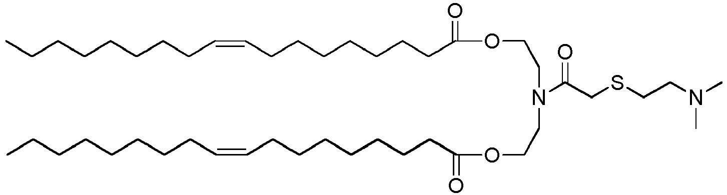 |
| | (Z)-((2-((2-(dimethylamino)ethyl)thio)acetyl)azanediyl)bis(ethane-2,1-diyl)dioleate |
| I10 | 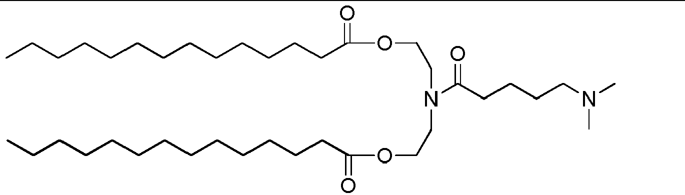 |
| | ((5-(dimethylamino)pentanoyl)azanediyl)bis(ethane-2,1-diyl)ditetradecanoate |
| I11 | 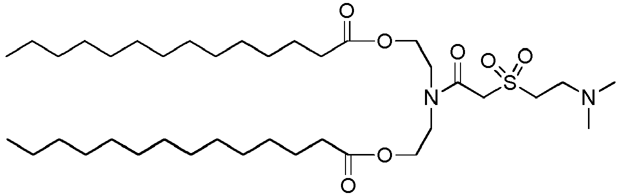 |
| | ((2-((2-(dimethylamino)ethyl)sulfonyl)acetyl)azanediyl)bis(ethane-2,1-diyl)ditetradecanoate |

**[Table 11]**

| | |
|---|---|
| I12 | 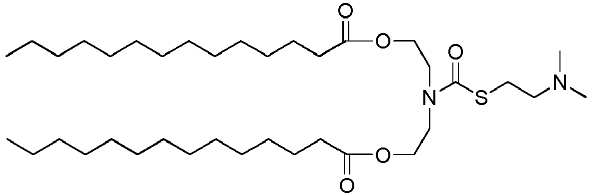 |
| | ((((2-(dimethylamino)ethyl)thio)carbonyl)azanediyl)bis(ethane-2,1-diyl)ditetradecanoate |
| I13 | 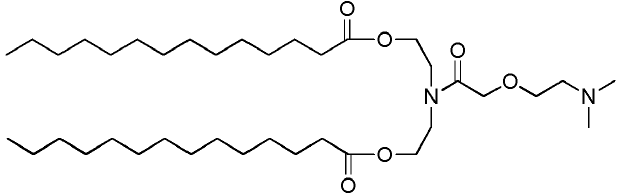 |
| | ((2-(2-(dimethylamino)ethoxy)acetyl)azanediyl)bis(ethane-2,1-diyl)ditetradecanoate |
| I14 | 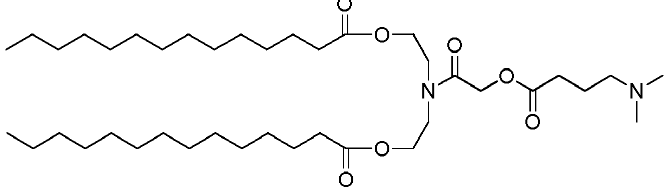 |
| | ((2-((4-(dimethylamino)butanoyl)oxy)acetyl)azanediyl)bis(ethane-2,1-diyl)ditetradecanoate |
| I15 | 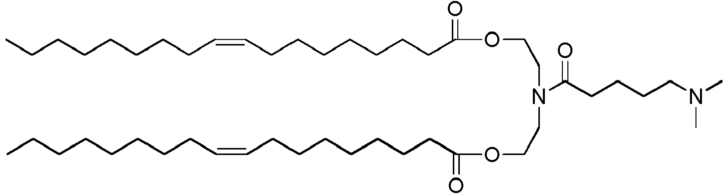 |
| | (Z)-((5-(dimethylamino)pentanoyl)azanediyl)bis(ethane-2,1-diyl)dioleate |
| I16 | 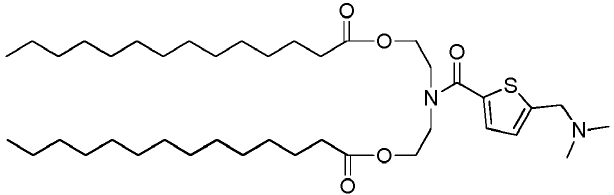 |
| | ((5-((dimethylamino)methyl)thiophene-2-carbonyl)azanediyl)bis(ethane-2,1-diyl)di-tetradec anoate |

**[Table 12]**

| | |
|---|---|
| I17 | 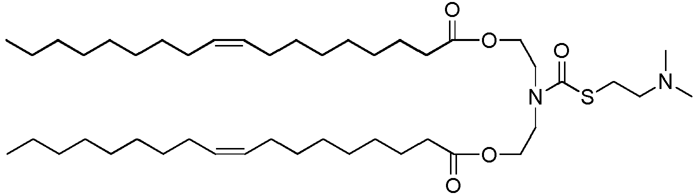 |
| | (Z)-((((2-(dimethylamino)ethyl)thio)carbonyl)azanediyl)bis(ethane-2,1-diyl)dioleate |
| I18 | 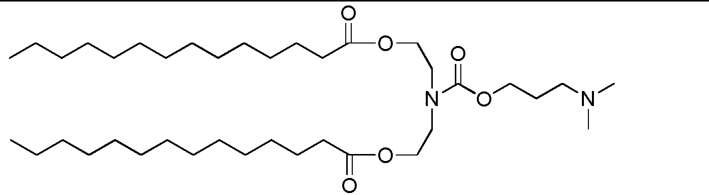 |
| | (((3-(dimethylamino)propoxy)carbonyl)azanediyl)bis(ethane-2,1-diyl)ditetradecanoate |
| I19 | 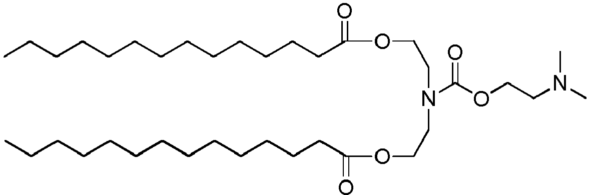 |
| | (((2-(dimethylamino)ethoxy)carbonyl)azanediyl)bis(ethane-2,1-diyl)ditetradecanoate |
| I20 | 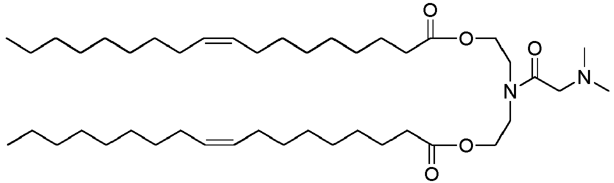 |
| | ((2-(dimethylamino)acetyl)azanediyl)bis(ethane-2,1-diyl)ditetradecanoate |
| I21 | 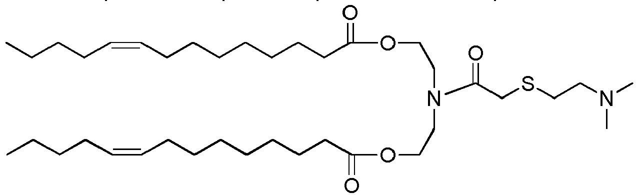 |
| | (9Z,9'Z)-((2-((2-(dimethylamino)ethyl)thio)acetyl)azanediyl)bis(ethane-2,1-diyl)bis(tetradec -9-enoate) |
| I22 | 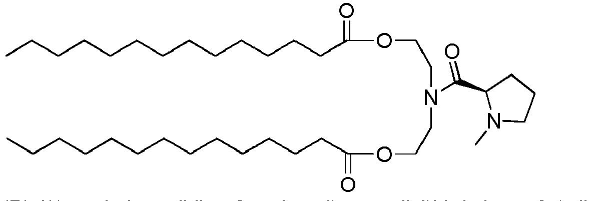 |
| | (R)-((1-methylpyrrolidine-2-carbonyl)azanediyl)bis(ethane-2,1-diyl)ditetradecanoate |

**[Table 13]**

| | |
|---|---|
| I23 | 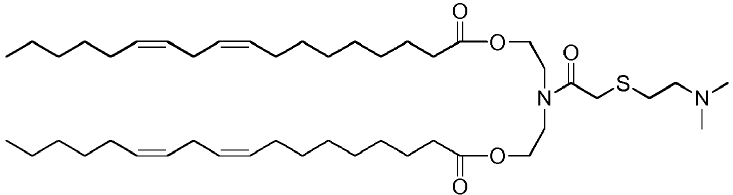 |
| | (9Z,9'Z,12Z,12'Z)-((2-((2-(dimethylamino)ethyl)thio)acetyl)azanediyl)bis(ethane-2,1-diyl)bi s(octadeca-9,12-dienoate) |
| I24 | 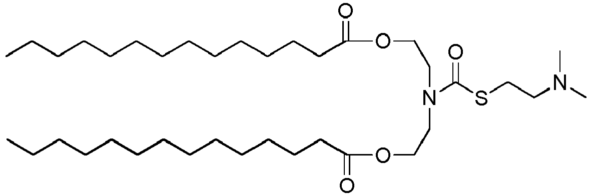 |
| | (9Z,9'Z,12Z,12'Z)-((2-((2-(dimethylamino)ethyl)thio)acetyl)azanediyl)bis(ethane-2,1-diyl)bi s(octadeca-9,12-dienoate) |

A production method for the above-mentioned lipids is described in WO 2012/170952 and WO 2013/185116, and a person skilled in the art can produce the lipids according to the description of these documents.

The carrier may contain one type or two or more types of lipids. In some embodiments, the carrier contains the always-charged cationic lipid and the ionizable cationic lipid. By blending the ionizable cationic lipid, an adverse effect of the always-charged cationic lipid can be reduced. In a specific embodiment, the carrier contains a lipid selected from the group consisting of a helper lipid, a PEG-lipid, and a sterol in addition to the always-charged cationic lipid and the ionizable cationic lipid. Non-limiting examples of a combination of lipids contained in the carrier include, for example, a combination of lipid P2 and lipid 18, a combination of lipid P2, lipid 18, DOPE, cholesterol, and PEG-DMPE, a combination of DODC, DOPE, cholesterol, and a PEG-lipid, a combination of lipid P9, DOPE, cholesterol, and a PEG-lipid, and a combination of DC-6-14, DOPE, and cholesterol. Non-limiting specific examples of the combination of lipids contained in the carrier include, for example, lipid P2 : lipid 18 (a molar ratio of 1:1), lipid P2 : lipid 18 : DOPE: cholesterol : PEG-DMPE (a molar ratio of 4:4:6:5:1), DODC : DOPE : cholesterol : a PEG-lipid (a molar ratio of 25:5:19:1), lipid P9 : DOPE : cholesterol : a PEG-lipid (a molar ratio of 25:5:19:1), and DC-6-14 : DOPE : cholesterol (a molar ratio of 4:3:3).

In one embodiment, the carrier constituent component other than the retinoid or the retinoid conjugate includes a polymer. Non-limiting examples of such a polymer include cationic polymers (polycations) such as polyethylene glycol (PEG), polyethyleneimine (PEI), and polylysine (e.g. poly-L-lysine (PLL)), non-cationic polymers such as polylactic acid (PLA), polyglycolic acid (PGA), and a lactic acid-glycolic acid copolymer (PLGA), and/or polymers based on derivatives thereof.

The carrier in the present disclosure may have a specific three-dimensional structure. Examples of such a structure include, but are not limited to, a straight-chain or branched linear structure, a film-like structure, and a spherical structure. Therefore, the carrier may have, but not limited to, any three-dimensional structure such as a dendrimer, a dendron, a micelle, a liposome, an emulsion, a microsphere, or a nanomicrosphere. Non-limiting examples of such a carrier are known from Marcucci and Lefoulon, Drug Discov Today 2004 Mar 1; 9(5): 219-28, Torchilin, Eur J Pharm Sci 2000 Oct; 11 Suppl 2: S81-91, and the like.

The carrier of the present disclosure may be cationic or non-cationic (e.g. anionic, nonionic, or electrically neutral) . In one embodiment, the carrier is cationic. In another embodiment, the carrier is electrically neutral (for example, has a zeta potential in a range of -10 mV to +10 mV) under physiological conditions, but becomes cationic under acidic conditions in an endosome or the like. The carrier of the present disclosure may have binding affinity to albumin and/or a low-density lipoprotein (LDL). Further, the carrier may be one capable of forming a lipoplex or a polyplex with a substance to be supported. The lipoplex can be formed, for example, between a cationic lipid and a substance having a negative charge (e.g. a nucleic acid such as DNA or RNA), and the polyplex can be formed, for example, between a polycation and a substance having a negative charge.

The carrier may be a lipid structural body. The lipid structural body means a structural body having any three-dimensional structure, for example, a linear shape, a film-like shape, a spherical shape, or the like, and containing a lipid as a constituent component, and includes, but is not limited to, a liposome, a micelle, a lipid microsphere, a lipid nanosphere, a lipid emulsion, and the like. The lipid structural body can be stabilized by, for example, adjusting an osmotic pressure using an osmotic pressure regulating agent such as a salt, a saccharide such as sucrose, glucose, or maltose, or a polyhydric alcohol such as glycerin or propylene glycol, preferably sucrose or glucose. Further, the pH may be adjusted by adding a pH adjusting agent such as a moderate salt or buffer solution. Therefore, the production, storage, or the like of the lipid structural body can be performed in a medium containing such a substance. In that case, the concentration of the osmotic pressure regulating agent is preferably adjusted to be isotonic with blood. For example, in the case of sucrose, the concentration thereof in a medium may be, but not limited to, from 3 to 15 wt%, preferably from 5 to 12 wt%, more preferably from 8 to 10 wt%, particularly preferably 9 wt%, and in the case of glucose, the concentration thereof in a medium may be, but not limited to, from 1 to 10 wt%, preferably from 3 to 8 wt%, more preferably from 4 to 6 wt%, particularly preferably 5 wt%.

The bonding or incorporation of the retinoid and/or the retinoid conjugate to or in the carrier of the present disclosure is also possible by bonding or incorporation of the retinoid and/or the retinoid conjugate to or in another constituent component of the carrier by a chemical and/or physical method. Alternatively, the bonding or incorporation of the retinoid and/or the retinoid conjugate to or in the carrier of the present disclosure is also possible by mixing the retinoid and/or the retinoid conjugate with the carrier constituent component other than this when producing the carrier. The amount of the retinoid and/or the retinoid conjugate in the carrier of the present disclosure can be set to, for example, 0.01 to 1000 nmol/µL, preferably 0.1 to 100 nmol/µL. Further, in the carrier of the present disclosure containing the retinoid and/or the retinoid conjugate and the carrier constituent component other than the retinoid and the retinoid conjugate, the molar ratio of the retinoid and/or the retinoid conjugate to the carrier constituent component other than the retinoid and the retinoid conjugate may be, but not limited to, for example, from 8:1 to 1:4 or from 4:1 to 1:2. The bonding or incorporation of the retinoid and/or the retinoid conjugate to or in the carrier may be performed before allowing the carrier to support a substance to be delivered, or may be performed by simultaneously mixing the carrier, the retinoid and/or the retinoid conjugate, and a substance to be delivered, or may be performed by mixing the carrier in a state where a substance to be delivered has already been supported thereon, and the retinoid and/or the retinoid conjugate, or the like. Therefore, the present disclosure also relates to a method for producing a preparation specific to extracellular matrix-producing cells in the skin, including a step of bonding the retinoid and/or the retinoid conjugate to any existing drug binding carrier or drug enclosing carrier, for example, a liposome preparation such as DaunoXome(R), Doxil, Caelyx(R), or Myocet(R).

The bonding or incorporation of the retinoid and/or the retinoid conjugate to or in the carrier of the present disclosure can also be realized by bonding or incorporating the retinoid and/or the retinoid conjugate to or in another component of the carrier by a chemical and/or physical method. Alternatively, the bonding or incorporation of the retinoid and/or the retinoid conjugate to or in the carrier of the present disclosure may be performed by mixing the retinoid and/or the retinoid conjugate having formation affinity and basic components of the carrier in the carrier components during the preparation of the carrier. The amount of the retinoid and/or the retinoid conjugate to be bonded or incorporated in the carrier of the present disclosure may be from 0.01% to 100%, preferably from 0.2% to 20%, more preferably from 1% to 5% in a weight ratio with respect to the carrier components.

The carrier of the present disclosure may contain, for example, an aqueous or nonaqueous gel, a cream, a multiple emulsion, a microemulsion, a liposome, an ointment, an aqueous or nonaqueous solution, a lotion, an aerosol, or a hydrocarbon base or powder, and can also contain an excipient such as a solubilizer, a permeation enhancer (e.g. a fatty acid, a fatty acid ester, a fatty alcohol, or an amino acid), and a hydrophilic polymer (e.g. polycarbophil or polyvinylpyrrolidone). In one embodiment, the pharmaceutically acceptable carrier is a liposome or a percutaneous absorption enhancer. Examples of a known liposome include the following:
- CellFectin, that is, a liposome preparation of a cationic lipid N,N^{I},N^{II},N^{III}-tetramethyl-N,N^{I},N^{II},N^{III}-tetrapalmitylspermine and dioleoylphosphatidylethanolamine (DOPE) at 1:1.5 (M/M) (GIBCO BRL),
- Cytofectin GSV, that is, a liposome preparation of a cationic lipid and DOPE at 2:1 (M/M) (Glen Research), DOTAP (N-[1-(2,3-dioleoyloxy)-N,N,N-trimethyl-ammoniummethylsulfa te] (Boehringer Manheim),
- Lipofectamine, that is, a liposome preparation of a polycationic lipid DOSPA, a neutral lipid DOPE, and a dialkylated amino acid (DiLA2) at 3:1 (M/M) (GIBCO BRL), and
- Lipotrust, that is, a liposome preparation of O,O'-ditetradecanoyl-N-(α-trimethylammonioacetyl)diethanol amine chloride (DC-6-14), cholesterol, and dioleoylphosphatidylethanolamine at 4:3:3 (M/M) (Hokkaido System Science).

The present disclosure also relates to a method for producing a carrier for delivering a substance to extracellular matrix-producing cells in the skin, including a step of blending a retinoid as a component for promoting the delivery of a substance to extracellular matrix-producing cells in the skin. As a method for blending the retinoid, for example, various methods described herein can be used. Therefore, the blending of the retinoid can be performed by bonding or incorporating the retinoid and/or the retinoid conjugate to or in another constituent component of the carrier by a chemical and/or physical method, or mixing the retinoid and/or the retinoid conjugate with a carrier constituent component other than this when producing the carrier. The blending amount or the like of the retinoid and/or the retinoid conjugate is as previously mentioned with respect to the carrier of the present disclosure .

The substance to be delivered by the carrier of the present disclosure is not particularly limited, but is preferably a substance having a size capable of physically moving within the body of an organism from an administration site to a lesion site where target cells are present. Therefore, the carrier of the present disclosure can carry not only a substance such as an atom, a molecule, a compound, a protein, or a nucleic acid, but also an object such as a vector, a virus particle, a cell, a drug release system constituted by one or more elements, or a micromachine. The substance to be delivered preferably has a property of affecting the target cells in some way, and includes, for example, a substance with which the target cells are labeled, and a substance that controls the activity or growth of the target cells (for example, enhances or suppresses this).

Therefore, in one embodiment of the present disclosure, the substance to be delivered by the carrier includes "a drug that controls the activity or growth of extracellular matrix-producing cells in the skin". Here, the activity of the extracellular matrix-producing cells in the skin refers to various activities such as secretion, uptake, and migration exhibited by the extracellular matrix-producing cells in the skin, and in the present disclosure, typically, it means an activity involved particularly in the onset, progression, and/or recurrence or the like of skin fibrosis among these. Examples of such an activity include, but without being limited to, the production or secretion of an extracellular matrix component such as collagen, proteoglycan, tenascin, fibronectin, thrombospondin, osteopontin, osteonectin, or elastin, or the like, and the suppression of decomposition activity of such an extracellular matrix component.

Therefore, in the present disclosure, the drug that controls the activity or growth of extracellular matrix-producing cells in the skin may be any drug that suppresses directly or indirectly the physical, chemical, and/or physiological action or the like of the cells involved in the onset, progression, and/or recurrence of skin fibrosis, and includes, but is not limited to, a substance that inhibits the production or secretion of the above-mentioned extracellular matrix component or the like, a cell growth inhibitor, an apoptosis inducer, a TIMP (Tissue inhibitor of metalloproteinase) inhibitor, and the like.

Examples of the substance that inhibits the production or secretion of an extracellular matrix component or the like include, but are not limited to, a substance such as an interfering nucleic acid, a ribozyme, or an antisense nucleic acid that suppresses the expression of an extracellular matrix component such as collagen, proteoglycan, tenascin, fibronectin, thrombospondin, osteopontin, osteonectin, or elastin, or a substance having a dominant negative effect such as a dominant negative mutant, a vector expressing such a substance, and cells transformed thereby. Among the extracellular matrix components, a drug that inhibits the production or secretion of collagen includes, without being limited to, for example, an inhibitor of HSP47 which is a collagen-specific molecular chaperone essential for intracellular transport and molecular maturation that are common to the synthetic processes of various types of collagen, for example, an HSP47 expression inhibitor such as an interfering nucleic acid, a ribozyme, or an antisense nucleic acid for HSP47, a substance having a dominant negative effect such as a dominant negative mutant of HSP47, a vector expressing such a substance, cells transformed thereby, and the like.

Examples of the cell growth inhibitor include, but are not limited to, alkylating agents such as ifosfamide, nimustine (e.g. nimustine hydrochloride), cyclophosphamide, dacarbazine, melphalan, and ranimustine, metabolism antagonists such as gemcitabine (e.g. gemcitabine hydrochloride), enocitabine, cytarabine ocfosfate, a cytarabine preparation, tegafur/uracil, a tegafur/gimeracil/oteracil potassium combination drug (e.g. TS-1), doxifluridine, hydroxycarbamide, fluorouracil, methotrexate, and mercaptopurine, antitumor antibiotics such as idarubicin (e.g. idarubicin hydrochloride), epirubicin (e.g. epirubicin hydrochloride), daunorubicin (e.g. daunorubicin hydrochloride and daunorubicin citrate), doxorubicin (e.g. doxorubicin hydrochloride), pirarubicin (e.g. pirarubicin hydrochloride), bleomycin (e.g. bleomycin hydrochloride), peplomycin (e.g. peplomycin sulfate), mitoxantrone (e.g. mitoxantrone hydrochloride), and mitomycin C, alkaloids such as etoposide, irinotecan (e.g. irinotecan hydrochloride), vinorelbine (e.g. vinorelbine tartrate), docetaxel (e.g. docetaxel hydrate), paclitaxel, vincristine (e.g. vincristine sulfate), vindesine (e.g. vindesine sulfate), and vinblastine (e.g. vinblastine sulfate), hormone therapy agents such as anastrozole, tamoxifen (e.g. tamoxifen citrate), toremifene (e.g. toremifene citrate), bicalutamide, flutamide, and estramustine (e.g. estramustine phosphate), platinum complexes such as carboplatin, cisplatin (CDDP), and nedaplatin, angiogenesis inhibitors such as thalidomide, neovastat, and bevacizumab, and L-asparaginase.

Examples of the apoptosis inducer include, but without being limited to, compound 861, gliotoxin, and atorvastatin.

Examples of the TIMP (e.g. TIMP1, TIMP2, TIMP3, or the like) inhibitor include, but without being limited to, a TIMP activity inhibitor such as an antibody for a TIMP, a TIMP production inhibitor such as an interfering nucleic acid, a ribozyme, or an antisense nucleic acid for a TIMP, a vector expressing the same, and cells transformed with the same.

Further, the "drug that controls the activity or growth of extracellular matrix-producing cells in the skin" in the present disclosure may be any drug that directly or indirectly promotes the physical, chemical, and/or physiological action or the like of extracellular matrix-producing cells in the skin involved directly or indirectly in suppression of the onset, progression, and/or recurrence of skin fibrosis.

The carrier of the present disclosure can deliver one type or two or more types of the above-mentioned drugs.

The interfering nucleic acid in the present disclosure includes double-stranded RNA such as siRNA (small interfering RNA), miRNA (micro RNA), shRNA (short hairpin RNA), piRNA (Piwi-interacting RNA), and rasiRNA (repeat associated siRNA) and modified forms thereof. The nucleic acid in the present disclosure includes RNA, DNA, PNA, and complexes thereof.

The nucleic acid may have various known modifications. By such a modification, a useful characteristic such as improvement of the stability of the nucleic acid can be provided. The modification may be a modification of various moieties of the nucleic acid, for example, one or two or more moieties such as a sugar, a nucleic acid base, a phosphate group, and a phosphodiester backbone. Non-limiting examples of a modified sugar moiety include 2'-O-methyl, 2'-methoxyethoxy, 2'-deoxy, 2'-fluoro, 2'-allyl, 2'-O-[2-(methylamino)-2-oxoethyl], 4'thio, 4'-(CH₂)₂-O-2' bridge, a 2' locked nucleic acid, and 2'-O-(N-methylcarbamate). Non-limiting examples of a modified nucleic acid base include xanthine, hypoxanthine, 2-amino-adenine, 6-methyl and other alkyl derivatives of adenine and guanine, 2-propyl and other alkyl derivatives of adenine and guanine, 5-halouracil and 5-halocytosine, 5-propynyluracil and 5-propynylcytosine, 6-azouracil, 6-azocytosine, and 6-azothymine, 5-uracil (pseudouracil), 4-thiouracil, 8-halo, amino, thiol, thioalkyl, hydroxy, and other 8-substituted adenines and guanines, 5-trifluoromethyl, and other 5-substituted uracils and cytosines, 7-methylguanine and an acyclonucleotide. Non-limiting examples of the modification to the phosphodiester backbone include a substitution of a phosphodiester bond with a phosphorothioate, 3'- (or -5') deoxy-3'- (or -5') thio-phosphorothioate, phosphorodithioate, phosphoroselenate, 3'- (or -5') deoxyphosphinate, boranophosphate, 3'- (or -5') deoxy-3'- (or 5'-) amino phosphoramidate, hydrogenphosphonate, boranophosphate ester, phosphoramidate, and alkyl or aryl phosphonate and phosphotriester, or phosphorus bond, or the like.

Additional examples of the substance to be delivered by the carrier of the present disclosure include, but are not limited to, a drug other than the above-mentioned drugs that suppress the onset, progression, and/or recurrence of skin fibrosis, and can include, for example, but without being limited to, TGF-β1 inhibitors (including an anti-TGF-β1 antibody, a TGF-β1 vaccine, etc.), pentoxifylline and a metabolite thereof, a calcium channel blocker (nifedipine, etc.), a prostanoid receptor agonist (iloprost, etc.), an ACE inhibitor (captopril, enalapril, etc.), an endothelin receptor inhibitor (bosentan, etc.), a PDE-5 inhibitor (sildenafil, etc.), interferon-y, a CD20 inhibitor (an anti-CD20 antibody, for example, rituximab, etc.), an anti-TNF-a antibody (infliximab, etc.), an mTOR inhibitor (sirolimus, everolimus, etc.), thalidomide, hydroxychloroquine, tacrolimus, and an immunosuppressant (a steroid, azathioprine, cyclophosphamide, methotrexate, cyclosporine, D-penicillamine, etc.). These drugs can also be used in combination with a composition of the present disclosure described later. Here, the expression "used in combination" includes substantially simultaneous administration of the composition of the present disclosure and the above-mentioned drug and administration at a temporal interval within the same treatment period. In the former case, the composition of the present disclosure may be administered by being mixed with the above-mentioned drug or may be continuously administered without being mixed therewith. In the latter case, the composition of the present disclosure may be administered before or after the administration of the above-mentioned drug.

In one embodiment of the present disclosure, examples of the drug that controls the activity or growth of extracellular matrix-producing cells in the skin include an HSP47 inhibitor, for example, an interfering nucleic acid and the like for HSP47. Non-limiting examples of the interfering nucleic acid for HSP47 are described in WO 2011/072082, and the like.

In some embodiments, the interfering nucleic acid for HSP47 is a double-stranded nucleic acid having a structure (A1):
5' (N)x-Z 3' (antisense strand)
3' Z'-(N')y-z" 5' (sense strand)
wherein each of N and N' is a nucleotide which may be unmodified or modified, or an unconventional moiety,
each of (N) x and (N')y is an oligonucleotide in which each consecutive N or N' is covalently linked to the next N or N',
each of Z and Z' is independently present or absent, but if present, independently includes 1 to 5 consecutive nucleotides or non-nucleotide moieties or a combination thereof covalently attached to the 3' end of the strand in which it is present,
z" may be present or absent, but if present, is a capping moiety covalently attached to the 5' end of (N')y,
each of x and y is independently an integer between 18 and 40,
the sequence of (N')y has complementarity to the sequence of (N)x, and (N)x includes an antisense sequence to mRNA encoding HSP47.

In a specific embodiment, the interfering nucleic acid for HSP47 is a double-stranded nucleic acid having a structure (A2) :
5' N¹-(N)x-Z 3' (antisense strand)
3' Z'-N²-(N')y-z" 5' (sense strand)
wherein each of N², N, and N' is an unmodified ribonucleotide or a modified ribonucleotide or an unconventional moiety,
each of (N) x and (N')y is an oligonucleotide in which each consecutive N or N' is linked to the adjacent N or N' through a covalent bond,
each of x and y is independently an integer between 17 and 39,
the sequence of (N')y has complementarity to the sequence of (N)x, and (N)x has complementarity to the consecutive sequence of mRNA encoding HSP47,
N¹ is covalently bonded to (N)x and is mismatched with the mRNA encoding HSP47 or is a complementary DNA moiety to the target RNA,
N¹ is a moiety selected from the group consisting of natural or modified uridine, deoxyribouridine, ribothymidine, deoxyribothymidine, adenosine, or deoxyadenosine,
z" may be present or absent, but if present, is a capping moiety covalently attached to the 5' end of N²-(N')y, and
each of Z and Z' is independently present or absent, but if present, is independently 1 to 5 consecutive nucleotides or consecutive non-nucleotide moieties or a combination thereof covalently attached to the 3' end of the strand in which it is present.

The unconventional moiety in the above-mentioned structures (A1)and (A2) includes, but is not limited to, for example, moieties selected from the group consisting of a non-nucleotide moiety (e.g., an abasic moiety, an inverted abasic moiety (an inverted abasic deoxyribose moiety, an inverted abasic ribose moiety, etc.), a hydrocarbon (alkyl) moiety (a non-nucleotide C3, C4, or C5 moiety, etc.), derivatives thereof, etc.), a deoxyribonucleotide, a modified deoxyribonucleotide, a mirror nucleotide (L-DNA, L-RNA, etc.), a non-base pairing nucleotide analog, a nucleotide linked to an adjacent nucleotide through a 2'5' internucleotide phosphate bond, a bridged nucleic acid (LNA, an ethylene bridged nucleic acid, etc.), a bond-modified nucleotide(PACE, etc.), and a base-modified nucleotide.

The capping moiety in the above-mentioned structures (A1) and (A2) includes a moiety that can be covalently linked to the 5' end of (N')y, and includes, but is not limited to, for example, an abasic ribose moiety, an abasic deoxyribose moiety, modifications of abasic ribose and abasic deoxyribose moieties (including 2' O alkyl modifications), inverted abasic ribose and abasic deoxyribose moieties and modifications thereof, C6-imino-Pi, C6-amino-Pi, a mirror nucleotide (L-DNA, L-RNA, etc.), a 5'O-Me nucleotide, and a nucleotide analog, for example, a 4',5'-methylene nucleotide, a 1-(β-D-erythrofuranosyl) nucleotide, a 4'-thionucleotide, a carbocyclic nucleotide, 5'-amino-alkyl phosphate, 1,3-diamino-2-propyl phosphate, 3-aminopropyl phosphate, 6-aminohexyl phosphate, 12-aminododecyl phosphate, hydroxypropyl phosphate, a 1,5-anhydrohexitol nucleotide, an alpha-nucleotide, a threo-pentofuranosyl nucleotide, an acyclic 3',4'-seco nucleotide, a 3,4-dihydroxybutyl nucleotide, a 3,5-dihydroxypentyl nucleotide, a 5'5'-inverted abasic moiety, 1,4-butylene glycol phosphate, 5'-amino, and bridging or non-bridging methylphosphonate, and a 5'-mercapto moiety.

The non-nucleotide moiety in the above-mentioned structures (A1) and (A2) includes, but is not limited to, for example, an abasic moiety, for example, a deoxyriboabasic moiety (herein sometimes referred to as "dAb") or a riboabasic moiety (herein sometimes referred to as "rAb"), two abasic moieties covalently linked to each other (preferably through a phosphoric acid-based bond) (e.g., dAb-dAb or rAb-rAb or dAb-rAb or rAb-dAb), and an alkyl moiety (arbitrarily, a propane [(CH₂)₃] moiety (C3) or a derivative thereof including propanol (C3OH) and propanediol ("C3Pi")). In some embodiments, the phosphoric acid-based bond includes a phosphorothioate, phosphonoacetate, or phosphodiester bond. In some embodiments, the non-nucleotide moiety includes a C2, C3, C4, C5, or C6 alkyl moiety, an arbitrarily, a C3 [propane, -(CH₂)₃-] moiety, or propanol (C3OH), propanediol, and a derivative thereof including a phosphodiester derivative of propanediol ("C3Pi") .

In various embodiments, the alkyl moiety includes an alkyl derivative containing a C3 alkyl, C4 alkyl, C5 alkyl, or C6 alkyl moiety containing a terminal hydroxyl group, a terminal amino group, or a terminal phosphate group. In some embodiments, the alkyl moiety is a C3 alkyl or C3 alkyl derivative moiety. In some embodiments, the C3 alkyl moiety includes propanol, propyl phosphate, propyl phosphorothioate, or a combination thereof. In some embodiments, the non-nucleotide moiety is selected from propyl phosphate, propyl phosphorothioate, propyl phospho-propanol, propyl phospho-propyl phosphorothioate, propyl phospho-propyl phosphate (propyl phosphate) 3, (propyl phosphate) 2-propanol, and (propyl phosphate) 2-propyl phosphorothioate.

In one embodiment, the mRNA encoding HSP47 in the above-mentioned structures (A1) and (A2) has a nucleotide sequence represented by SEQ ID NO: 1.

Non-limiting examples of a combination of the antisense strand and the sense strand in the structure (A1) are shown in Table 5.

### [Table 14]

**Table 5. Non-limiting examples of a combination of the antisense strand and the sense strand in the structure (A1)**

| Nucleic acid NOS | Sense strand (5'>3') | SEQ ID NOS | Antisense strand (5'>3') | SEQ ID NOS |
|---|---|---|---|---|
| A1-1 | GAGACACAUGGGUGCUAUU | 2 | AAUAGCACCCAUGUGUCUC | 3 |
| A1-2 | ACAAGAUGCGAGACGAGUU | 4 | AACUCGUCUCGCAUCUUGU | 5 |
| A1-3 | CCUUUGACCAGGACAUCUA | 6 | UAGAUGUCCUGGUCAAAGG | 7 |
| A1-4 | CGGACAGGCCUCUACAACU | 8 | AGUUGUAGAGGCCUGUCCG | 9 |
| A1-5 | ACUCCAAGAUCAACUUCCA | 10 | UGGAAGUUGAUCUUGGAGU | 11 |
| A1-6 | GACAAGAUGCGAGACGAGU | 12 | ACUCGUCUCGCAUCUUGUC | 13 |
| A1-7 | CCUGAGACACAUGGGUGCU | 14 | AGCACCCAUGUGUCUCAGG | 15 |

Non-limiting examples of a combination of the antisense strand and the sense strand in the structure (A2) are shown in Table 6.

### [Table 15]

**Table 6. Non-limiting examples of a combination of the antisense strand and the sense strand in the structure (A2)**

| Nucleic acid NOS | Sense strand (5'>3') | SEQ ID NOS | Antisense strand (5'>3') | SEQ ID NOS |
|---|---|---|---|---|
| A2-1 | GAGACACAUGGGUGCUAUA | 16 | UAUAGCACCCAUGUGUCUC | 17 |
| A2-2 | ACAAGAUGCGAGACGAGUA | 18 | UACUCGUCUCGCAUCUUGU | 19 |
| A2-3 | CGGACAGGCCUCUACAACA | 20 | UGUUGUAGAGGCCUGUCCG | 21 |
| A2-4 | UGACAAGAUGCGAGACGAA | 22 | UUCGUCUCGCAUCUUGUCA | 23 |
| A2-5 | ACUCCAAGAUCAACUUCCU | 24 | AGGAAGUUGAUCUUGGAGU | 25 |
| A2-6 | UCCUGAGACACAUGGGUGA | 26 | UCACCCAUGUGUCUCAGGA | 27 |
| A2-7 | GACAAGAUGCGAGACGAGA | 28 | UCUCGUCUCGCAUCUUGUC | 29 |
| A2-8 | ACAGGCCUCUACAACUACA | 30 | UGUAGUUGUAGAGGCCUGU | 31 |

In more specific embodiments, the nucleic acid (in the structure (A1), x=y=19, and in the structure (A2), x=y=18) having the structure (A1) and/or (A2) includes the following combinations of modifications.

Combination 1: the sense strand includes 5 consecutive 2'5' ribonucleotides at positions 15, 16, 17, 18, and 19 (5'>3') at the 3' end, a C3Pi moiety covalently attached to the 3' end, and an inverted abasic moiety covalently attached to the 5' end, and the antisense strand includes 2'OMe sugar modified ribonucleotides at positions 1, 3, 5, 9, 11, 13, 15, 17, and 19 (5'>3'), a 2'5' ribonucleotide at position 7, and a C3Pi-C3OH moiety covalently attached to the 3' end.

Combination 2: the sense strand includes 2'OMe sugar modified ribonucleotides at positions 2, 14, and 18 (5'>3'), a C3OH moiety covalently attached to the 3' end, and an inverted abasic deoxyribonucleotide moiety covalently attached to the 5' end, and the antisense strand includes 2'OMe sugar modified ribonucleotides at positions 1, 3, 5, 9, 12, 13, and 17 (5'>3'), a 2'5' ribonucleotide at position 7, and a C3Pi-C3OH moiety covalently attached to the 3' end.

Combination 3: the sense strand includes 2'5' ribonucleotides at positions 15, 16, 17, 18, and 19 (5'>3'), a C3OH moiety covalently attached to the 3' end, and an inverted abasic deoxyribonucleotide moiety covalently attached to the 5' end, and the antisense strand includes 2'OMe sugar modified ribonucleotides at positions 2, 4, 6, 8, 11, 13, 15, 17, and 19 (5'>3'), a 2'5' ribonucleotide at position 7, and a C3Pi-C3OH moiety covalently attached to the 3' end.

Combination 4: the sense strand includes 2'OMe sugar modified ribonucleotides at positions 4, 11, 13, and 17 (5'>3'), a 2'5' ribonucleotide at position 9, a C3OH moiety covalently attached to the 3' end, and an inverted abasic deoxyribonucleotide moiety covalently attached to the 5' end, and the antisense strand includes 2'OMe sugar modified ribonucleotides at positions 1, 4, 8, 11, and 15 (5'>3'), a 2'5' ribonucleotide at position 6, and a C3Pi-C3OH moiety covalently attached to the 3' end.

Combination 5: the sense strand includes 2'OMe sugar modified ribonucleotides at positions 7, 13, 16, and 18 (5'>3'), a 2'5' ribonucleotide at position 9, a C3OH moiety covalently attached to the 3' end, and an inverted abasic moiety covalently attached to the 5' end, and the antisense strand includes 2'OMe sugar modified ribonucleotides at positions 1, 3, 5, 9, 11, 13, 15, 17, and 19 (5'>3'), a 2'5' ribonucleotide at position 7, and a C3Pi-C3OH moiety covalently attached to the 3' end.

Combination 6: the sense strand includes 5 consecutive 2'5' ribonucleotides at positions 15, 16, 17, 18, and 19 (5'>3'), a C3Pi moiety covalently attached to the 3' end, and an inverted abasic moiety covalently attached to the 5' end, and the antisense strand includes 2'OMe sugar modified ribonucleotides at positions 1, 3, 5, 9, 11, 13, 15, 17, and 19 (5'>3'), a 2'5' ribonucleotide at position 7, and a C3Pi-C3OH moiety covalently attached to the 3' end.

Combination 7: the sense strand includes 2'OMe sugar modified ribonucleotides at positions 2, 14, and 18 (5'>3'), a C3OH moiety covalently attached to the 3' end, and an inverted abasic deoxyribonucleotide moiety covalently attached to the 5' end, and the antisense strand includes 2'OMe sugar modified ribonucleotides at positions 1, 3, 5, 9, 11, 13, and 17 (5'>3'), a 2'5' ribonucleotide at position 7, and a C3Pi-C3OH moiety covalently attached to the 3' end.

Combination 8: the sense strand includes 2'OMe sugar modified ribonucleotides at positions 4, 11, 13, and 17 (5'>3'), a C3OH moiety covalently attached to the 3' end, and an inverted abasic deoxyribonucleotide moiety covalently attached to the 5' end, and the antisense strand includes 2'OMe sugar modified ribonucleotides at positions 1, 4, 8, 13, and 15 (5'>3'), a 2'5' ribonucleotide at position 6, and a C3Pi-C3OH moiety covalently attached to the 3' end.

Combination 9: the sense strand includes 2'OMe sugar modified ribonucleotides at positions 2, 4, 11, 13, and 17 (5'>3'), a C3OH moiety covalently attached to the 3' end, and an inverted abasic deoxyribonucleotide moiety covalently attached to the 5' end, and the antisense strand includes 2'OMe sugar modified ribonucleotides at positions 1, 4, 8, 11, and 15 (5'>3'), a 2'5' ribonucleotide at position 6, and a C3Pi-C3OH moiety covalently attached to the 3' end.

In the above-mentioned combinations, the C3Pi moiety represents propyl phosphate, the C3OH moiety represents propanol, and the C3Pi-C3OH moiety represents propyl phospho-propanol. In a specific embodiment, the above-mentioned respective moieties have the following structures. The phosphate group at the left end represents a phosphate group bonded to the 3' end of a nucleic acid. Further, in the present disclosure, the "3' end" of a nucleic acid means an end on the opposite side to the 5' end. Therefore, for example, when a nucleotide at an end on the opposite side to the 5' end has a phosphate group capable of further bonding to a chemical moiety not at position 3', but at position 2' such a chemical moiety is actually bonded to the 2' end of the nucleic acid, however, in the present disclosure, even in such a case, it is expressed for convenience as bonded to the 3' end.

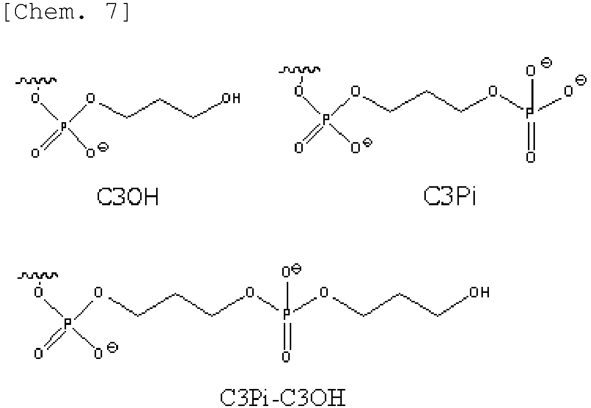

In a specific embodiment, the interfering nucleic acid for HSP47 has the following structure.

### Structure 1:

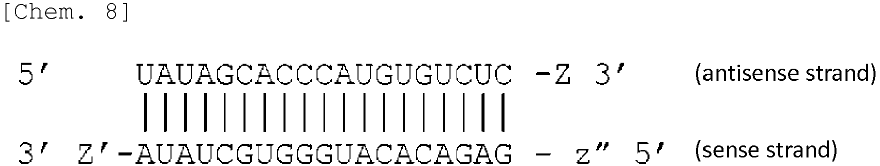

In the above-mentioned structure, the antisense strand (SEQ ID NO: 17) includes 2'OMe sugar modified ribonucleotides at positions 1, 3, 5, 9, 11, 15, 17, and 19 (5'>3'), and a 2' 5' ribonucleotide at position 7, and Z is a C3Pi-C3OH moiety, and the sense strand (SEQ ID NO: 16) includes 2'5' ribonucleotides at positions 15, 16, 17, 18, and 19 (5'>3'), Z' is a C3Pi moiety, and z" is an inverted abasic deoxyribonucleotide moiety.

### Structure 2:

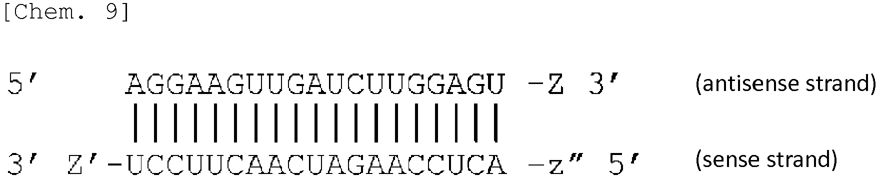

In the above-mentioned structure, the antisense strand (SEQ ID NO: 25) includes 2'OMe sugar modified ribonucleotides at positions 2, 4, 6, 8, 11, 13, 15, 17, and 19 (5'>3') and a 2'5' ribonucleotide at position 7, and Z is C3Pi-C3OH, and the sense strand (SEQ ID NO: 24) includes 2'5' ribonucleotides at positions 15, 16, 17, 18, and 19 (5'>3'), Z' is a C3OH moiety, and z" is an inverted abasic deoxyribonucleotide moiety.

### Structure 3:

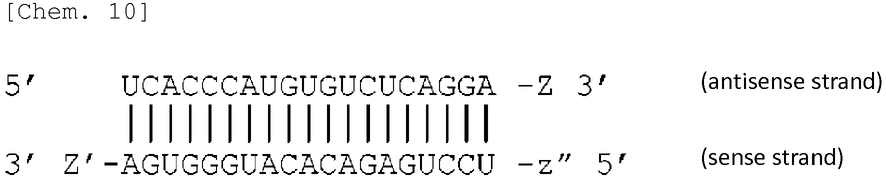

In the above-mentioned structure, the antisense strand (SEQ ID NO: 27) includes 2'OMe sugar modified ribonucleotides at positions 1, 4, 8, 11, and 15 (5'>3'), and a 2'5' ribonucleotide at position 6, and Z is a C3Pi-C3OH moiety, and the sense strand (SEQ ID NO: 26) includes 2'OMe sugar modified ribonucleotides at positions 4, 11, 13, and 17 (5'>3'), a 2'5' ribonucleotide at position 9, Z' is a C3OH moiety, and z" is an inverted abasic deoxyribonucleotide moiety.

The substance or object to be delivered by the carrier of the present disclosure may or may not be labeled. Labeling enables monitoring of the success or failure of delivery to target cells, or the increase or decrease of target cells, etc., and is particularly useful not only at the test/research level but also at the clinical level. A label may be selected from any label known to a person skilled in the art, for example, any radioisotope, magnetic material, gas or substance that generates a gas under physiological conditions, element that exhibits nuclear magnetic resonance (e.g. hydrogen, phosphorus, sodium, fluorine, etc.), substance that affects the relaxation time of an element exhibiting nuclear magnetic resonance (e.g. a metal atom or a compound containing a metal atom), substance that binds to a labeled substance (e.g. an antibody, etc.), fluorescent substance, fluorophore, chemiluminescent substance, biotin or derivative thereof, avidin or derivative thereof, enzyme, etc. The label may be attached to a carrier constituent component or may be supported on a carrier as an independent substance to be delivered.

In the present disclosure, the expression "for extracellular matrix-producing cells in the skin" or "for delivery to extracellular matrix-producing cells in the skin" means that it is suitable to use the extracellular matrix-producing cells in the skin as target cells, and this includes, for example, that it is possible to deliver a substance to the cells, more rapidly, more highly efficiently, and/or in a larger amount than to other cells, for example, normal cells. For example, the carrier of the present disclosure can deliver a substance to the extracellular matrix-producing cells in the skin at a rate and/or efficiency of 1.1 times or more, 1.2 times or more, 1.3 times or more, 1.5 times or more, 2 times or more, or even 3 times or more compared with other cells. In the present disclosure, the "deliver" includes a series of processes including a step of allowing target cells to incorporate a substance to be delivered and a step of allowing the substance to be delivered incorporated in the cells to reach a site of action, and the promotion of delivery means promotion of any one or more of these steps. The step of allowing target cells to incorporate a substance to be delivered includes, but is not limited to, incorporation of a substance to be delivered by endocytosis, pinocytosis, or the like. The step of allowing the substance to be delivered incorporated in the cells to reach a site of action includes, but is not limited to, endosomal escape, transfer to a specific intracellular site (e.g., a cell nucleus, a cytoplasm, a cell membrane, a mitochondrion, etc.), or the like.

The promotion of delivery can be evaluated by comparing the behavior of the substance to be delivered in the individual steps of delivery or the action of the substance to be delivered as a final result of the delivery with that by a similar carrier which does not contain a component for promoting the delivery, or the like. For example, the incorporation into the cells can be evaluated by observing the change over time of the position of the substance to be delivered having a detectable label attached thereto, or the like, and the endosomal escape can be evaluated by observing the change over time of the positional relationship between the substance to be delivered having a detectable label attached thereto and the endosome in a cell in which the endosome is specifically stained, or the like. Further, the action of the substance to be delivered can be evaluated by a method according to the substance to be delivered. For example, when the substance to be delivered is an inhibitory nucleic acid against a specific gene, the action thereof can be evaluated by determining the expression level of the gene. The promotion degree is not particularly limited, and may be a promotion of 1.1 times or more, 1.2 times or more, 1.3 times or more, 1.5 times or more, 2 times or more, or even 3 times or more compared with a similar carrier which does not contain a component for promoting the delivery.

The present disclosure also relates to a composition for controlling the activity or growth of extracellular matrix-producing cells in the skin, for treating skin fibrosis or fibrogenesis due to GVHD, or for regenerating a normal skin tissue from a fibrotic skin tissue, the composition containing the above-mentioned carrier and the above-mentioned drug that controls the activity or growth of extracellular matrix-producing cells in the skin, and also relates to use of the carrier in the production of such a composition. One embodiment of the composition of the present disclosure contains a retinoid in an effective amount for promoting the delivery of a substance to extracellular matrix-producing cells in the skin. Further, in one embodiment of the composition of the present disclosure, the delivery of a substance to extracellular matrix-producing cells in the skin is promoted by a retinoid.

The skin fibrosis in the present disclosure refers to a pathological condition characterized by excessive accumulation of an extracellular matrix (e.g., collagen, etc.) in the skin, and includes, but is not limited to, for example, abnormal scarring (hypertrophic scars, keloids, etc.) associated with all possible types of accidental and iatrogenic (surgical) skin injuries (traumatic injuries, surgical wounds, thermal injuries (including chemical burns, burns caused by heat, or radiation burns), skin grafting, etc.), skin diseases such as scleroderma (systemic scleroderma, macular scleroderma, etc.), skin GVHD, Dupuytren's contracture, epidermolysis bullosa, skin fibrosis induced by a drug (e.g., taxane, bleomycin, etc.), and restrictive dermopathy, skin fibrogenesis involved in a disease such as porphyria cutanea tarda, nephrogenic systemic fibrosis, eosinophilia myalgia syndrome, or toxic oil syndrome, and the like. Fibrogenesis due to GVHD in the present disclosure includes fibrogenesis not only in the skin, but also in various organs (lung, liver, etc.).

Skin GVHD is a skin manifestation involved in GVHD and is often developed in a subject having cGVHD. GVHD is a pathological condition that may occur by attacking a tissue (particularly, a normal tissue) of a recipient by white blood cells or the like of a donor after allogeneic hematopoietic stem cell transplantation performed as a part of a treatment for a neoplastic disease such as hematologic malignancy (e.g., leukemia such as acute myeloid leukemia, acute promyelocytic leukemia, acute lymphoblastic leukemia, chronic myeloid leukemia, or chronic lymphocytic leukemia, lymphoma such as Hodgkin's lymphoma or non-Hodgkin's lymphoma, multiple myeloma, etc.) or a solid tumor (particularly, a metastatic solid tumor), myelodysplastic syndrome (MDS), aplastic anemia, or the like, and can be lethal when it becomes serious. On the other hand, GVHD is also a phenomenon indicating a success of colonization of transplanted donor cells. In a treatment of a tumor involving hematopoietic stem cell transplantation, before transplantation, generally, a treatment of a tumor with radiation and/or a chemotherapeutic agent, or the like (a pretransplant conditioning regimen) is performed. It can be said that the pretransplant conditioning regimen is desirably performed so as to eradicate the target tumor as much as possible, however, in an elderly subject (a human subject aged, for example, 55 years or older, 60 years or older, 65 years or older, 70 years or older, 75 years or older, or the like), a subject affected with another complication (e.g., diabetes, a kidney disease, a heart disease, etc.), or the like, a high-intensity conditioning regimen such as a myeloablative conditioning regimen imposes a great burden, and therefore, a lower-intensity conditioning regimen such as a non-myeloablative conditioning (NMAC) regimen or a reduced-intensity conditioning (RIC) regimen is applied in some cases.

When such a low-intensity conditioning regimen is applied to a neoplastic disease, there is a high possibility that tumor cells are not eradicated, and therefore, a graft-versus-leukemia (GVL) effect or a graft-versus-tumor (GVT) effect in which transplanted donor cells attack and eliminate residual tumor cells becomes important. Therefore, it is particularly beneficial for a subject to which a low-intensity conditioning regimen is applied to suppress the adverse effect of GVHD without impairing the GVL/GVT effect as much as possible. It has been demonstrated that the composition of the present disclosure does not significantly affect immune cells derived from a donor which is the origin of the GVL/GVT effect, and therefore, the composition of the present disclosure is particularly useful for a treatment of GVHD (particularly skin GVHD) after a low-intensity conditioning regimen or a treatment of a subject developing GVHD (particularly skin GVHD) after a low-intensity conditioning regimen, etc. In one embodiment of the present disclosure, skin fibrosis is GVHD after a low-intensity conditioning regimen, particularly skin GVHD after a low-intensity conditioning regimen. In a more specific embodiment, skin fibrosis is GVHD after a low-intensity conditioning regimen for a neoplastic disease, particularly skin GVHD after a low-intensity conditioning regimen for a neoplastic disease.

In one embodiment, the present disclosure relates to a composition for treating skin GVHD containing the above-mentioned carrier and the above-mentioned drug that controls the activity or growth of extracellular matrix-producing cells in the skin. In some embodiments, the composition is a composition for treating skin GVHD caused by allogeneic hematopoietic stem cell transplantation after a low-intensity conditioning regimen. In a specific embodiment, the composition is a composition for treating skin GVHD caused by allogeneic hematopoietic stem cell transplantation after a low-intensity conditioning regimen in an elderly person or a patient affected with a complication. In another specific embodiment, the composition is a composition for treating skin GVHD caused by allogeneic hematopoietic stem cell transplantation after a low-intensity conditioning regimen without significantly affecting immune cells derived from a donor. In another specific embodiment, the composition is a composition for treating skin GVHD caused by allogeneic hematopoietic stem cell transplantation after a low-intensity conditioning regimen for a neoplastic disease. In a more specific embodiment, the composition is a composition (i) for treating skin GVHD caused by allogeneic hematopoietic stem cell transplantation after a low-intensity conditioning regimen in an elderly person or a patient affected with a complication without significantly affecting immune cells derived from a donor, (ii) for treating skin GVHD caused by allogeneic hematopoietic stem cell transplantation after a low-intensity conditioning regimen for a neoplastic disease in an elderly person or a patient affected with a complication, (iii) for treating skin GVHD caused by allogeneic hematopoietic stem cell transplantation after a low-intensity conditioning regimen for a neoplastic disease without significantly affecting immune cells derived from a donor, or (iv) for treating skin GVHD caused by allogeneic hematopoietic stem cell transplantation after a low-intensity conditioning regimen for a neoplastic disease in an elderly person or a patient affected with a complication without significantly affecting immune cells derived from a donor.

In another embodiment, the present disclosure relates to a composition for treating fibrogenesis involved in GVHD containing the above-mentioned carrier and the above-mentioned drug that controls the activity or growth of extracellular matrix-producing cells in the skin. In some embodiments, the composition is a composition for treating fibrogenesis involved in GVHD caused by allogeneic hematopoietic stem cell transplantation after a low-intensity conditioning regimen. In a specific embodiment, the composition is a composition for treating fibrogenesis involved in GVHD caused by allogeneic hematopoietic stem cell transplantation after a low-intensity conditioning regimen in an elderly person or a patient affected with a complication. In another specific embodiment, the composition is a composition for treating fibrogenesis involved in GVHD caused by allogeneic hematopoietic stem cell transplantation after a low-intensity conditioning regimen without significantly affecting immune cells derived from a donor. In another specific embodiment, the composition is a composition for treating fibrogenesis involved in GVHD caused by allogeneic hematopoietic stem cell transplantation after a low-intensity conditioning regimen for a neoplastic disease. In a more specific embodiment, the composition is a composition (i) for treating fibrogenesis involved in GVHD caused by allogeneic hematopoietic stem cell transplantation after a low-intensity conditioning regimen in an elderly person or a patient affected with a complication without significantly affecting immune cells derived from a donor, (ii) for treating fibrogenesis involved in GVHD caused by allogeneic hematopoietic stem cell transplantation after a low-intensity conditioning regimen for a neoplastic disease in an elderly person or a patient affected with a complication, (iii) for treating fibrogenesis involved in GVHD caused by allogeneic hematopoietic stem cell transplantation after a low-intensity conditioning regimen for a neoplastic disease without significantly affecting immune cells derived from a donor, or (iv) for treating fibrogenesis involved in GVHD caused by allogeneic hematopoietic stem cell transplantation after a low-intensity conditioning regimen for a neoplastic disease in an elderly person or a patient affected with a complication without significantly affecting immune cells derived from a donor. In these embodiments, fibrogenesis involved in GVHD may also occur in a tissue other than the skin, for example, the liver, the lung, etc. Further, the composition for treating fibrogenesis involved in GVHD may be a preparation for systemic administration (e.g., a preparation for intravenous administration, etc.).

In the present disclosure, the expression "regenerating a normal skin tissue from a fibrotic skin tissue" means that a skin tissue denatured by fibrogenesis is restored at least to a state where fibrogenesis is of a milder degree, more preferably to a state before fibrogenesis occurs. That is, as skin fibrogenesis progresses, a skin tissue is replaced with a fibrous tissue which is mainly an extracellular matrix, however, the regeneration of a normal skin tissue from a fibrotic skin tissue in the present disclosure is such that the above flow is reversed, and the proliferated fibrous tissue is replaced with an original normal tissue. Therefore, the regeneration of a normal skin tissue from a fibrotic skin tissue in the present disclosure includes not only complete restoration of a fibrotic skin tissue to the original state, but also partial restoration of a fibrotic skin tissue to the original state. The degree of regeneration of a normal skin tissue may be evaluated by histological examination of a biopsy sample or the like based on normalization of the tissue structure, a reduction in a region occupied by fibrous tissues, an increase in a region occupied by normal tissues, or the like, or when an abnormality of a biochemical index or the like due to fibrogenesis is observed before a treatment with the present composition, evaluation may be performed based on improvement or the like of the index or the like.

In the composition of the present disclosure, the carrier may include a substance to be delivered in the inside thereof or may be present in a state of being adhered to the outside of a substance to be delivered, or may be mixed with a substance to be delivered. Therefore, according to the route of administration, the drug release manner, or the like, the above-mentioned composition may be coated with an appropriate material, for example, an enteric coating or a timed disintegration material, or may be incorporated into an appropriate drug release system. Further, the composition of the present disclosure may be in the form of a complex of a lipid carrier containing a retinoid and a substance to be delivered, that is a lipoplex. Further, when the carrier is constituted by only a retinoid and/or a retinoid conjugate, the composition of the present disclosure may be in the form of a complex of a drug that controls the activity or growth of extracellular matrix-producing cells in the skin and a retinoid and/or a retinoid conjugate.

The composition of the present disclosure can be used as a medicine (that is, a pharmaceutical composition) and may be administered through various routes including both oral and parenteral routes, for example, but without being limited to, through a route such as an oral, intravenous, intramuscular, subcutaneous, local, intrapulmonary, tracheobronchial, intratracheal, intrabronchial, nasal, transgastric, enteral, intrarectal, intraarterial, intraportal, intraventricular, intramedullary, intra-lymph node, intralymphatic, intracerebral, intrathecal, intracerebroventricular, transmucosal, percutaneous, intranasal, intraperitoneal, or intrauterine route, and it may be formulated into a dosage form suitable for each route of administration. As such a dosage form and a formulation method, any known form and method can be appropriately adopted.

For example, examples of the dosage form suitable for oral administration include, but are not limited to, a powder, a granule, a tablet, a capsule, a liquid, a suspension, an emulsion, a gel, and a syrup, and examples of the dosage form suitable for parenteral administration include injections such as an injectable solution, an injectable suspension, an injectable emulsion, and an injection to be prepared before use.

The preparation for parenteral administration can be in the form of an aqueous or nonaqueous isotonic aseptic solution or suspension. Examples of the dosage form suitable for percutaneous administration include an ointment, a cream, an emulsion, a plaster, and a cataplasm. A preparation for percutaneous administration may contain a component useful for percutaneous administration such as a percutaneous absorption enhancer.

The present disclosure also relates to a method for producing a pharmaceutical composition for treating skin fibrosis or fibrogenesis due to GVHD or a composition for regenerating a normal skin tissue from a fibrotic skin tissue, including a step of blending a retinoid as a component for promoting the delivery of a substance to extracellular matrix-producing cells in the skin and a drug that controls the activity or growth of the extracellular matrix-producing cells in the skin as an active ingredient. A method for blending a retinoid and/or a retinoid conjugate is not particularly limited as long as the retinoid and/or the retinoid conjugate in the composition in which the retinoid and/or the retinoid conjugate is blended can function as a component for promoting the delivery of a substance to extracellular matrix-producing cells in the skin, however, for example, various methods described herein can be used. Further, also a method for blending the active ingredient is not particularly limited as long as the active ingredient can exhibit a predetermined effect, and any known method can be used. The blending of the active ingredient may be performed simultaneously with the retinoid and/or the retinoid conjugate, or may be performed before or after blending the retinoid. For example, when the composition contains a carrier constituent component other than the retinoid and the retinoid conjugate, the blending of the active ingredient may be performed by mixing or the like of the carrier in which the retinoid and/or the retinoid conjugate has already been blended as the component for promoting the delivery of a substance with the active ingredient, or may be performed by simultaneously mixing or the like of the retinoid and/or the retinoid conjugate, the carrier constituent component other than the retinoid and the retinoid conjugate, and the active ingredient, or may be performed by, after blending the active ingredient in the carrier constituent component other than the retinoid and the retinoid conjugate, mixing or the like of the resulting material with the retinoid and/or the retinoid conjugate.

The blending amount or the like of the retinoid is as previously described with respect to the carrier of the present disclosure. Further, the blending amount of the active ingredient when it is administered as the composition may be an amount capable of suppressing the onset or recurrence of skin fibrosis or fibrogenesis due to GVHD, improving the pathological condition, alleviating the symptoms, or delaying or stopping the progression, preferably an amount capable of preventing the onset and recurrence of skin fibrosis or fibrogenesis due to GVHD or curing this, or an amount capable of regenerating a normal skin tissue from a fibrotic skin tissue. Further, it is preferably an amount that does not cause an adverse effect exceeding benefits from administration. Such an amount is known or can be appropriately determined by an in vitro test using cultured cells or the like or by a test in a model animal such as a mouse, a rat, a dog, or a pig, and such test methods are well known to a person skilled in the art. Examples of the model animal of skin fibrosis, for example, as a scleroderma model include those described in Asano et al, Curr Rheumatol Rep (2013) 15: 382 (e.g., a ROS (reactive oxygen species)-induced SS (systemic sclerosis) model, a Topo I (DNA topoisomerase I) and CFA (complete Freund's adjuvant)-induced SS model, an Ang II (angiotensin II)-induced SS model, a Fra-2 (Fos-related antigen 2) transgenic mouse, and a Fli-1 (Friend leukemia virus integration 1)^{ΔCTA(c-terminal activation domain)/ΔCTA} model, etc.). Examples of the model animal of GVHD involving skin GVHD include, but are not limited to, a minor histocompatibility antigen mismatch transplant model (transplantation of myeloid cells, spleen cells, or the like from a B10.D2 mouse to a BALB/c mouse) . The blending amount of the active ingredient can vary depending on the dosing form of the composition. For example, when the composition of a plurality of units is used for one administration, the amount of the active ingredient to be blended in one unit of the composition can be determined by dividing the amount of the active ingredient necessary for one administration by the plurality of units. Adjustment of such a blending amount can be performed appropriately by a person skilled in the art. For example, a dose level of about 0.1 mg to about 140 mg or so per kilogram of body weight per day is useful for the treatment for the above-mentioned state (about 0.5 mg to about 7 g per subject per day). The amount of an active component that can be combined with a carrier material for forming a single dosing form varies depending on a host to be treated and a specific administration method. The dosing unit form generally contains about 1 mg to about 500 mg of the active component.

It is understood that a specific dose level for an arbitrary specific subject depends on various factors including the specific activity of a compound to be used, the age, the body weight, the general health conditions, the gender, the diet, administration, the timing of administration, the route of administration, the rate of excretion, a medicine used in combination, and the severity of a specific disease under treatment.

The pharmaceutical composition according to the present disclosure can be administered once a day, qid, tid, bid, or at medically appropriate arbitrary intervals for an arbitrary period. However, a therapeutic agent can also be administered in a dosing unit including 2, 3, 4, 5, 6, or 7 sub-doses to be administered at appropriate intervals through a day. In that case, the active ingredient to be contained in each sub-dose may be set further smaller correspondingly so as to achieve the total daily dosing unit. Further, the dosing units can be combined for a single dose over several days using, for example, a conventional controlled-release preparation that brings about continuous constant release of the active ingredient over several days. The controlled-release preparation is well known in this technical field. The dosing unit may contain a corresponding plurality of daily doses. In the composition, the dosing units can be combined so that the sum of a plurality of units of the active ingredient together contains a sufficient dose.

The carrier or the composition of the present disclosure may be provided in a lyophilized state. When the carrier or the composition of the present disclosure is lyophilized, a lyophilization protecting agent may be utilized. Non-limiting examples of the lyophilization protecting agent include polysaccharides such as sucrose, lactulose, lactose, maltose, trehalose, cellobiose, kojibiose, nigerose, isomaltose, sophorose, laminaribiose, gentiobiose, turanose, maltulose, palatinose, gentiobiulose, mannobiose, melibiose, melibulose, rutinose, rutinulose, and xylobiose.

The carrier or the composition of the present disclosure can also be provided in a form that can be prepared before use, for example, a form that can be prepared by a doctor and/or a pharmacist, a nurse, or another paramedical, or the like in a medical care site or the vicinity thereof. The lyophilized form is one example of the form that can be prepared before use. In that case, the carrier or the composition of the present disclosure is provided as one or two or more containers containing at least one of the constituent elements essential therefor, and preparation is performed before use, for example, within before 24 hours, preferably within before 3 hours, more preferably immediately before use. When performing the preparation, a reagent, a solvent, a preparation instrument, or the like that is generally available in a preparation place can be appropriately used.

Therefore, the present disclosure also relates to a kit for preparing the carrier or the composition including one or two or more containers containing a retinoid and/or a retinoid conjugate, and/or a substance to be delivered, and/or a carrier constituent substance other than the retinoid and the retinoid conjugate singly or in combination, and necessary constituent elements for the carrier or the composition to be provided in the form of such a kit. The kit of the present disclosure may include other than the above-mentioned members, an instruction associated with a preparation method or an administration method for the carrier and the composition of the present disclosure, a treatment for skin fibrosis or fibrogenesis due to GVHD, or the like, for example, an instruction manual, an electronic recording medium such as a CD or a DVD, or the like. Further, the kit of the present disclosure may include all the constituent elements for completing the carrier or the composition of the present disclosure, but may not necessarily include all the constituent elements. Therefore, the kit of the present disclosure may not include a reagent, a solvent, for example, sterile water, physiological saline, a glucose solution, or the like that is generally available in a medical care site, an experiment facility, or the like.

The present disclosure further relates to a method for controlling the activity or growth of extracellular matrix-producing cells in the skin, for treating skin fibrosis or fibrogenesis due to GVHD, or for regenerating a normal skin tissue from a fibrotic skin tissue, the method including a step of administering an effective amount of the composition to a subject in need thereof. Here, the effective amount is, for example, in the latter case, an amount that suppresses the onset or recurrence of skin fibrosis or fibrogenesis due to GVHD, improves the pathological condition, alleviates the symptoms, or delays or stops the progression, preferably an amount that prevents the onset and recurrence of skin fibrosis or fibrogenesis due to GVHD or cures this, or may be an amount capable of regenerating a normal skin tissue from a fibrotic skin tissue. Further, it is preferably an amount that does not cause an adverse effect exceeding benefits from administration. Such an amount can be appropriately determined by an in vitro test using cultured cells or the like or by a test in a model animal such as a mouse, a rat, a dog, or a pig, and such test methods are well known to a person skilled in the art. Further, the dose of the retinoid contained in the carrier and the drug to be used in the method of the present disclosure is known to a person skilled in the art or can be appropriately determined by the above-mentioned test or the like. The model animal of skin fibrosis or GVHD is as described above.

A specific dose of the composition to be administered in the method of the present disclosure can be determined in consideration of various conditions with respect to the subject in need of a treatment, for example, the severity of the symptoms, the general health conditions of the subject, the age, the body weight, and the gender of the subject, the diet, the route of administration, the timing and frequency of administration, a medicine used in combination, the responsiveness to the treatment, compliance with the treatment, etc. As the route of administration, there are various routes including both oral and parenteral administration, and examples thereof include oral, intravenous, intramuscular, subcutaneous, local, intrapulmonary, tracheobronchial, intratracheal, intrabronchial, nasal, transgastric, enteral, intrarectal, intraarterial, intraportal, intraventricular, intramedullary, intra-lymph node, intralymphatic, intracerebral, intrathecal, intracerebroventricular, transmucosal, percutaneous, intranasal, intraperitoneal, and intrauterine routes.

The frequency of administration varies depending on the properties of the composition to be used and the conditions of the subject as described above, and may be, for example, a plurality of times per day (that is, 2, 3, 4 times, or 5 or more times per day), once a day, every few days (that is, every 2, 3, 4, 5, 6, or 7 days, etc.), a few times per week (e.g. 2, 3, 4 times, etc. per week), every week, or every few weeks (that is, every 2, 3, 4 weeks, etc.).

In the method of the present disclosure, the term "subject" means any living individual, preferably an animal, more preferably a mammal, further more preferably a human individual. In the present disclosure, the subject may be healthy or may be affected with some disease, but when a treatment of skin fibrosis is contemplated, it typically means a subject affected with skin fibrosis or having a risk of being affected with skin fibrosis. For example, when prevention of skin fibrosis is contemplated, typical examples include, but without being limited to, a subject affected with a disease causing skin fibrosis such as GVHD, porphyria cutanea tarda, nephrogenic systemic fibrosis, eosinophilia myalgia syndrome, or toxic oil syndrome.

The term "treatment" shall include medically acceptable all types of preventive and/or therapeutic interventions aiming at cure, temporary remission, or prevention of a disease, or the like. For example, the term "treatment" includes medically acceptable interventions aiming at various purposes including delay or stop of the progression of skin fibrosis, regression or elimination of a lesion, prevention of the onset or prevention of the recurrence of skin fibrosis, and the like.

In one embodiment, the subject in the method of the present disclosure is a subject having undergone a low-intensity conditioning regimen and having undergone or possibly undergoing subsequent allogeneic bone marrow stem cell transplantation. In a specific embodiment, such a subject is an elderly subject (a human subject aged, for example, 55 years or older, 60 years or older, 65 years or older, 70 years or older, 75 years or older, or the like) and/or a subject affected with another complication (e.g., diabetes, a kidney disease, a heart disease, etc.). In another specific embodiment, such a subject is affected with a neoplastic disease (e.g., hematopoietic malignancy, etc.). In more specific embodiment, such a subject is an elderly subject affected with a neoplastic disease and/or a subject affected with a neoplastic disease and a complication other than this.

In some embodiments, the method of the present disclosure further includes a step of identifying a subject having skin fibrosis or GVHD, or having a risk of developing this. In one embodiment, the method of the present disclosure further includes a step of performing allogeneic hematopoietic stem cell transplantation in a subject. In a specific embodiment, the method of the present disclosure further includes a step of performing a low-intensity conditioning regimen in a subject and a step of performing allogeneic hematopoietic stem cell transplantation in the subject.

The present disclosure further relates to a method for delivering a substance to extracellular matrix-producing cells in the skin utilizing the above-mentioned carrier. This method includes, but is not limited to, for example, a step of allowing the above-mentioned carrier to support a substance to be delivered, and a step of administering or adding the carrier having the substance to be delivered supported thereon to an organism or a medium, for example, a culture medium or the like containing extracellular matrix-producing cells in the skin. These steps can be appropriately achieved according to any known method, a method described herein, or the like. The above-mentioned delivery method can also be combined with another delivery method, for example, another delivery method targeting the skin, or the like. Further, the above-mentioned method also includes an embodiment performed in vitro and an embodiment targeting extracellular matrix-producing cells in the skin in vivo. The substance that can be transported by the carrier of the present disclosure is as described above.

### [Examples]

### Example 1: Preparation of siRNA-Containing Retinoid-Conjugated Liposomes

siRNA-containing retinoid-conjugated liposomes containing siRNA and a retinoid conjugate XR3 were prepared as described in WO 2012/170952. First, lipid P2, lipid 18, DOPE, cholesterol, PEG-DMPE, and XR3 were solubilized in anhydrous ethanol at a molar ratio of 20:20:30:25:5:2, whereby an ethanol/lipid mixture was obtained. siRNA was solubilized in a 50 mM citrate buffer solution, and the temperature was adjusted to 35 to 40°C. Subsequently, the ethanol/lipid mixture was added to the siRNA-containing buffer solution while stirring, whereby siRNA-containing liposomes were formed. The addition of the ethanol/lipid mixture was continued until the final ratio of the total lipids : siRNA reached 15:1 (w:w). Subsequently, the formed siRNA-containing liposomes were diafiltered against 10 times volume of PBS (pH 7.2), whereby ethanol was removed. The obtained liposomes (sometimes abbreviated as "XR3(+) liposomes" or "siRNA-containing retinoid-conjugated liposomes") had an average particle diameter of 50 to 100 nm, a PDI < 0.2, and showed a siRNA encapsulation efficiency > 85%. Further, when the zeta potential was measured, it was electrically neutral in a neutral pH range.

The sequences of the used siRNA are shown below.
siRNA 1
   Sense strand: 5'-GAGACACAUGGGUGCUAUA-3' (SEQ ID NO: 16)
   Antisense strand: 5'-UAUAGCACCCAUGUGUCUC-3' (SEQ ID NO: 17)
siRNA 2
   Sense strand: 5'-UCCUGAGACACAUGGGUGA-3' (SEQ ID NO: 26)
   Antisense strand: 5'-UCACCCAUGUGUCUCAGGA-3' (SEQ ID NO: 27) Scrambled siRNA
   Sense strand: 5'-CGAUUCGCUAGACCGGCUUCAUUGCAG-3' (SEQ ID NO: 32)
   Antisense strand: 5'-GCAAUGAAGCCGGUCUAGCGAAUCGAU-3' (SEQ ID NO: 33)

### Example 2: Inhibition of Expression of HSP47 in Dermal Myofibroblasts

In order to confirm that the siRNA-containing retinoid-conjugated liposomes can inhibit the expression of HSP47 in dermal myofibroblasts that are considered to be involved in skin fibrogenesis, the siRNA-containing retinoid-conjugated liposomes prepared in Example 1 were allowed to act on dermal myofibroblasts prepared in vitro, and the expression of HSP47 was measured. The dermal myofibroblasts were prepared by inducing dermal fibroblasts with TGF-β1. First, a skin piece of an untreated BALB/c mouse was digested with DMEM containing 5 mg/mL of collagenase type IV (Sigma-Aldrich) for 1 hour, and cultured in DMEM supplemented with 10% FCS for 2 to 3 days. The thus obtained primary dermal fibroblasts were cultured in DMEM supplemented with 10% FCS for 24 hours, and thereafter placed in an FCS-free medium for 12 hours, and recombinant human TGF-β1 (R&D Systems) was added thereto in the presence or absence of the siRNA-containing retinoid-conjugated liposomes at a siRNA concentration of 50 nM. After 24 hours, the cells were recovered in ISOGEN II (Nippon Gene), and RNA was extracted. A cDNA library was prepared using ReverTra Ace^{(R)} qPCR RT Master Mix with gDNA Remover (Toyobo) . By using TaqMan^{(R)} Fast Universal PCR Master Mix (Life Technologies), quantitative PCR (fluorescence probe method) of HSP47 was performed. The respective sequences of the used fluorescence probe (P) and primers (F/R) were as follows. Incidentally, the 5' end of the fluorescence probe is labeled with FAM.
serpinh1-P: 5'-AGCCACACTGGGATGAGAAGTTTCACCA-3' (SEQ ID NO: 34)
serpinh1-F: 5'-CTGCTTGTGAACGCCATGTTC-3' (SEQ ID NO: 35)
serpinh1-R: 5'-TCACCATGAAGCCACGGTTG-3' (SEQ ID NO: 36)

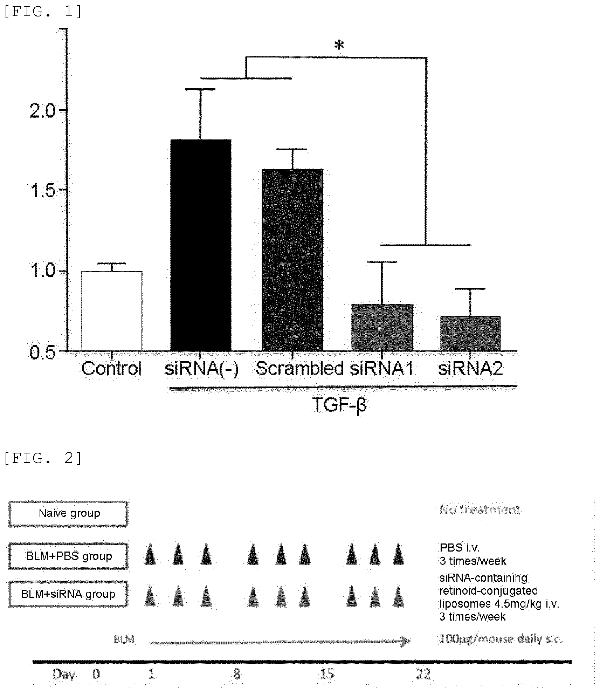

From the results shown in FIG. 1, it is found that both siRNA 1 and siRNA 2 significantly suppress the expression of HSP47 in the dermal myofibroblasts.

### Example 3: Treatment of Bleomycin-Induced Skin Fibrogenesis

According to a regimen shown in FIG. 2, a dorsal region of a C57BL/6 mouse was shaved, and bleomycin (BLM) was subcutaneously injected into the same region at a dose of 2 mg/kg every day over 21 days. In a treated group, from one day after the start of administration of bleomycin, the siRNA-containing retinoid-conjugated liposomes at a dose of 4.5 mg/kg·BW was intravenously injected 3 times per week (BLM+siRNA group) . As the siRNA, siRNA 1 was used. In the control group, a vehicle (PBS) was administered (BLM+PBS group).

On day 22 from the start of the experiment, the degree of skin fibrogenesis was evaluated according to the following (1) to (6).

### (1) Evaluation of Dermal Thickening by Skin Fibrogenesis:

The skin was collected from the dorsal region of each mouse and fixed overnight with 4% paraformaldehyde, and thereafter, paraffin embedding and section preparation were performed. The degree of fibrogenesis was evaluated by Masson's-Trichrome (MT) staining. The dermal thickness was measured at 5 sites per sample and averaged, and evaluation of dermal thickening (skin thickness) by fibrogenesis was performed.

### (2) Collagen Dot density:

An image of a portion with a length of 3 mm of the MT-stained sample was imported into an image analysis software Image Scope. By using an analysis program of Image Scope, the sum of color vector values at fibrotic sites was calculated.

### (3) Collagen Assay:

Skin samples obtained by perforating the skin in a circle (area: 19.6 mm²) with a diameter of 5 mm using a skin perforator (dermal punch 5 mm, Maruho Co., Ltd.) collected from 2 sites per individual were analyzed together. The skin samples were homogenized in 2 mL of 0.5 M acetic acid + 0.1 mg/mL of pepsin using TissueRuptor (Qiagen), and reacted at 4°C for 48 hours, and thereafter, the amount of collagen was quantitatively determined using Sircol Collagen Assay Kit (Biocolor) and GloMax^{(R)}-Multi Luminescence System (Promega).

### (4) Hydroxyproline Assay

The skin sample was placed in 6 N HCl and reacted at 110°C for 24 hours, followed by neutralization with 6 N NaOH. The resulting material was reacted with a Chloramin T solution for 20 minutes, and a color was developed with an Ehrlich solution, and then, an absorbance was measured.

### (5) qPCR Method from mRNA Extraction

The skin sample was instantaneously frozen with liquid nitrogen. Extraction of RNA and qPCR were performed in the same manner as in Example 1. Incidentally, in addition to HSP47, the expression of Col1a1 was also examined. The respective sequences of the fluorescence probe (P) for Col1a1 and the primers (F/R) were as follows. Incidentally, the 5' end of the fluorescence probe is labeled with FAM.
col1a1-P: 5'-CGGGGTCGGAGCCCTCGCTTCC-3' (SEQ ID NO: 37)
col1a1-F: 5'-ACCGATGGATTCCCGTTCGA-3' (SEQ ID NO: 38)
col1a1-R: 5'-CATTAGGCGCAGGAAGGTCAG-3' (SEQ ID NO: 39)

### (6) Immunofluorescent Staining:

Paraffin sections were prepared and stained using an anti-HSP47 antibody (abcam) and an anti-rabbit IgG antibody Alexa Fluor 594 as a secondary antibody.

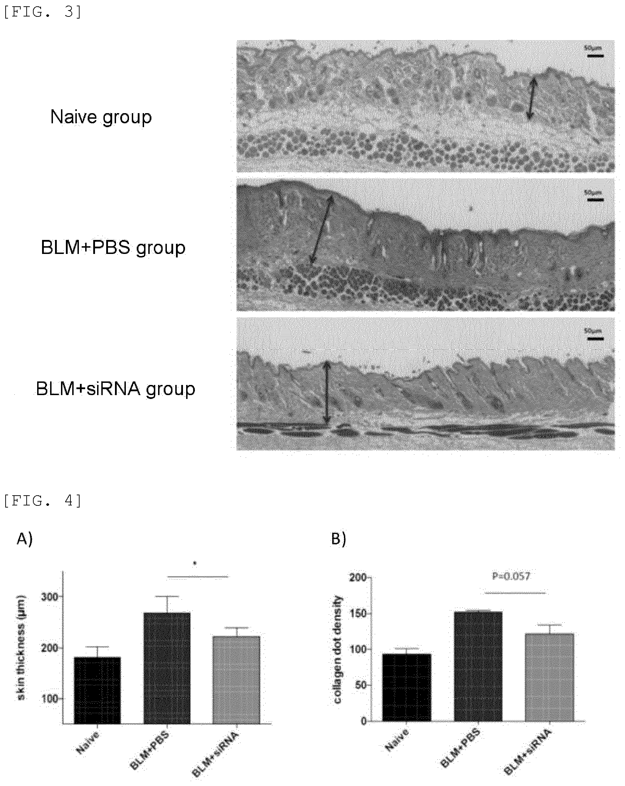

In the mice subjected to subcutaneous injection with bleomycin for 21 days, marked fibrogenesis occurred in the administration region (FIG. 3), and an increase in dermal thickening and collagen deposition (measured by collagen dot density) was observed (FIG. 4). However, as shown in FIG. 4, in the BLM+siRNA group in which the siRNA-containing retinoid-conjugated liposomes were administered along with BLM, as compared with the BLM+PBS group, dermal thickening was significantly suppressed, and also collagen deposition tended to be suppressed (p=0.057).

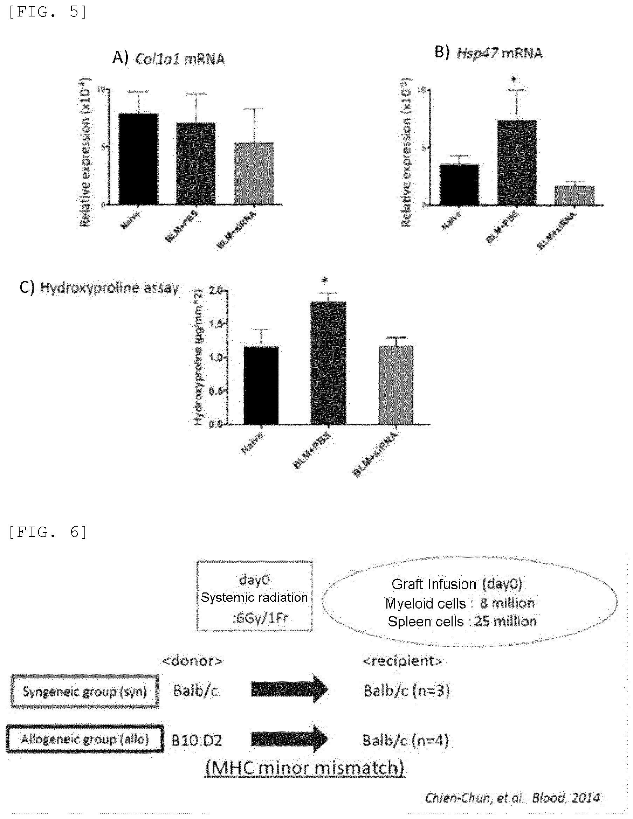

Further, when the expression of HSP47 was observed by the qPCR method, it was found that the expression is increased by administration of bleomycin, but in the BLM+siRNA group, the expression is markedly decreased (FIG. 5B). On the other hand, a significant change was not observed in the expression of Col1a1 (FIG. 5A).

When the amount of hydroxyproline in the skin was quantitatively determined, it was found that this is also increased by administration of bleomycin, but it is normalized by administration of the siRNA (FIG. 5C).

From these results, it was proved that in skin fibrogenesis, myofibroblasts produce collagen in a HSP47 dependent manner. Further, it was shown that the siRNA-containing retinoid-conjugated liposome is an effective fibrogenesis inhibitor in skin fibrosis.

### Example 4: Treatment of Skin GVHD 1

As shown in FIG. 6, after recipient mice (BALB/c) were subjected to systemic radiation (6 Gy), 8×10⁶ myeloid cells and 2.5×10⁷ spleen cells collected from a donor mouse (B10.D2) whose major histocompatibility complex (MHC) is matched and only minor histocompatibility antigen is mismatched were infused, whereby an allogeneic transplantation group (Allo group) was formed. As a control, to BALB/c mice subjected to systemic radiation in the same manner, myeloid cells and spleen cells collected from the same BALB/c mice were transplanted, whereby a syngeneic transplantation group (Syn group) was formed.

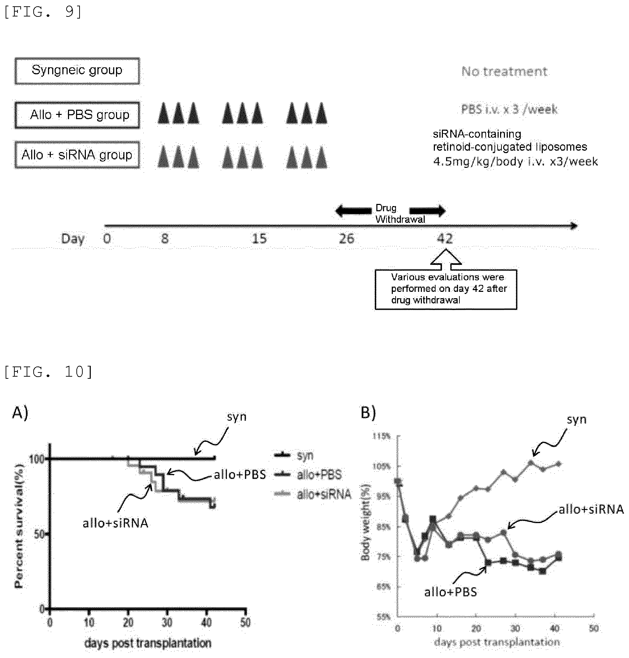

According to a regimen shown in FIG. 9, in a half of the recipients in the Allo group, the siRNA-containing retinoid-conjugated liposomes at a dose of 4.5 mg/kg·BW were intravenously injected 3 times per week from day 8 to day 26 after transplantation, whereby a treated group (Allo+siRNA group) was formed. As the siRNA, siRNA 1 was used. To the rest of the recipients in the Allo group, only PBS was administered as a control (Allo+PBS group).

The severity of skin fibrogenesis in the mice surviving on day 42 was evaluated according to the above (1) to (6).

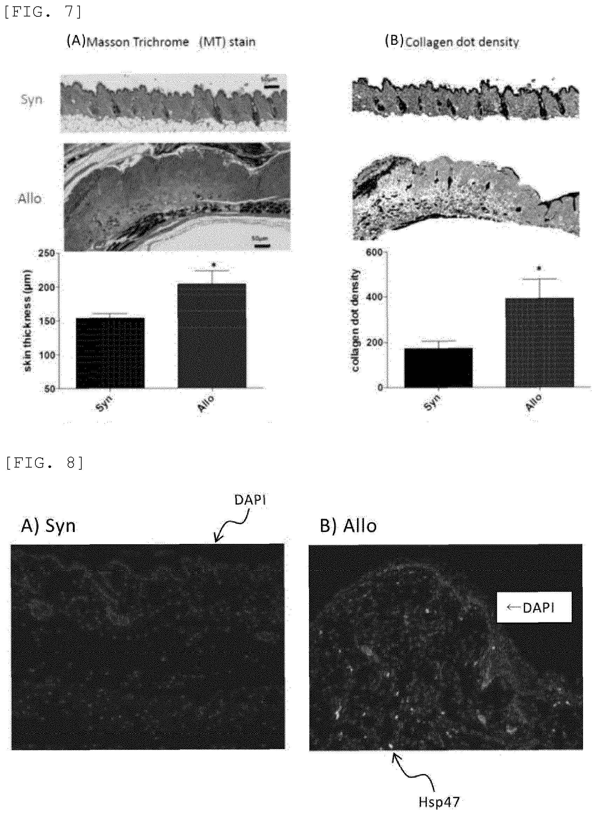

After BALB/c serving as the recipient mice were subjected to radiation, myeloid cells and spleen cells from a B10.D2 mouse serving as the donor were transplanted (FIG. 6). When fibrogenesis in the skin was evaluated after 6 weeks (on day 42), as compared with the Syn group in which syngeneic transplantation was performed as the control, dermal thickening and collagen deposition were significantly increased (FIG. 7) .

Further, by immunofluorescent staining of the skin sections, it was proved that in contrast with the Syn group, HSP47-positive cells were markedly increased in the Allo group (FIG. 8).

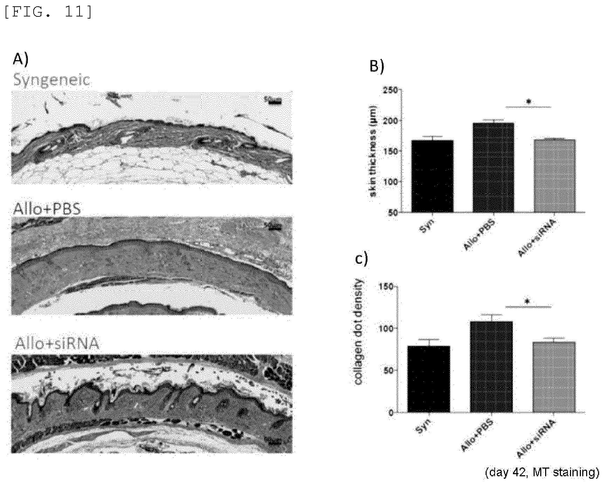

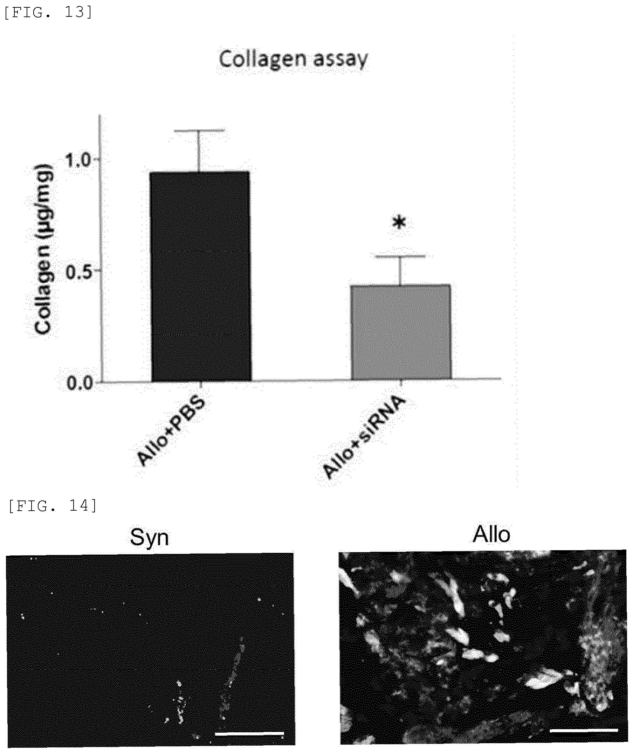

When the siRNA-containing retinoid-conjugated liposomes were administered 3 times per week from day 8 to day 26 after transplantation (FIG. 9), in the skin on day 42, dermal thickening and collagen deposition were decreased as compared with the Allo+PBS group serving as the control (FIG. 11). Also in quantitative determination of collagen by the collagen assay, in the siRNA-containing retinoid-conjugated liposome administration group (Allo+siRNA group), the amount of collagen in the skin was significantly decreased as compared with the Allo+PBS group (FIG. 13).

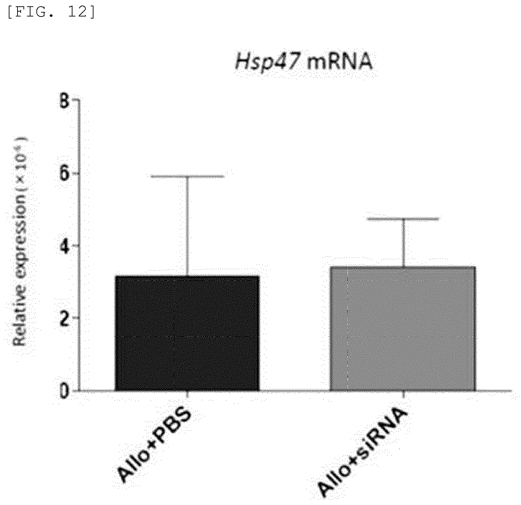

Although qPCR of HSP47 was performed, the expression of HSP47 was not suppressed in the siRNA-containing retinoid-conjugated liposome administration group (FIG. 12). This is considered to be because the administration of the siRNA-containing retinoid-conjugated liposomes was withdrawn for 16 days before the analysis (FIG. 9).

### Example 5: Treatment of Skin GVHD 2

An experiment was performed in the same manner as in Example 4 except that as the siRNA, siRNA 2 was used, and administration of the siRNA-containing retinoid-conjugated liposomes or the vehicle (PBS) was continued from day 1 to day 41. Further, in addition to the evaluation of skin fibrogenesis, the types of cells contained in the spleen and the thymus, the ratio thereof, etc. were analyzed by flow cytometry. More specifically, the thymus and the spleen were collected on day 42, and cell suspensions were prepared, and thereafter, red blood cells were lysed using RBC lysis buffer (Biolegend), and the number of viable cells was counted after trypan blue staining. The cell suspension from the thymus was stained with an anti-CD4 antibody and an anti-CD8 antibody, and double-positive cells were quantitatively determined by the flow cytometry method. In the cell suspension from the spleen, CD11b⁻ TCRb⁺ CD4⁺ cells and CD11b⁻ TCRb⁺ CD8⁺ cells were quantitatively determined as CD4⁺ T cells and CD8⁺ T cells, respectively by the flow cytometry method. A chimerism analysis was performed by staining the cell suspension from the spleen with an anti-Ly9.1 antibody and determining the ratio of recipient cells (Ly9.1 positive) by the flow cytometry method. Incidentally, in immunofluorescent staining, the skin in a dorsal region was collected on day 42 and a paraffin section was prepared, followed by immunostaining with an anti-HSP47 antibody (Abcam) and/or an anti-α-SMA antibody (Abeam), and nuclear staining with DAPI.

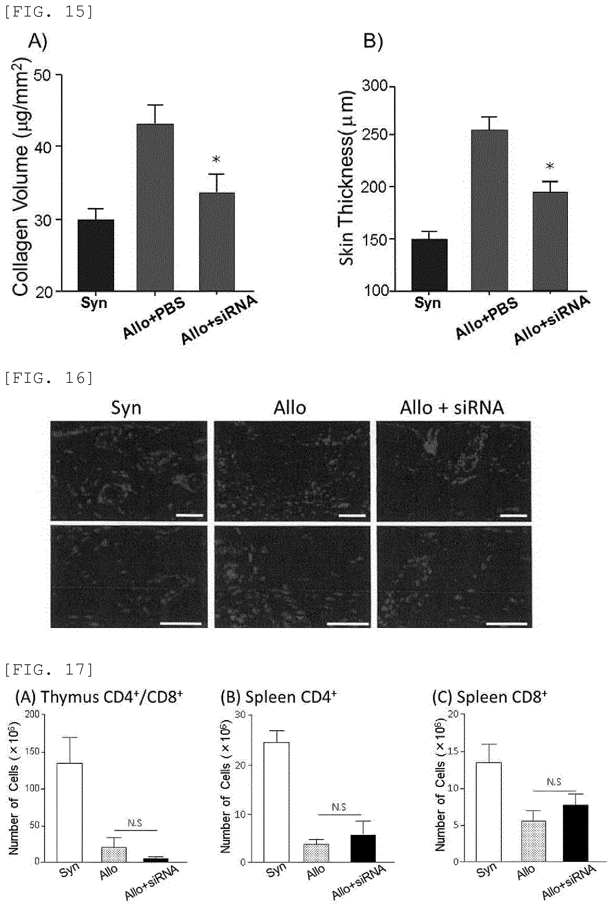

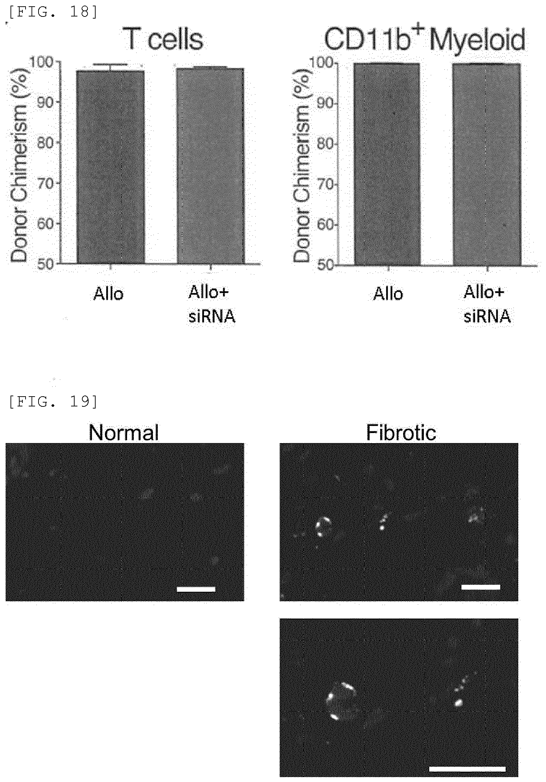

In the Allo group, occurrence of cGVHD was observed on around day 20. From the results shown in FIG. 14, it is found that on day 42, in the dermis in the Allo group, the number of α-SMA-positive myofibroblasts is increased as compared with the Syn group, and the cells were mainly accumulated in an avascular region of the dermis. Incidentally, α-SMA-positive pericytes were always present side by side with the vascular wall. The myofibroblasts also expressed HSP47. Further, from the results of evaluation of skin fibrogenesis shown in FIG. 15, in the same manner as in Example 3, it is found that in the siRNA-containing retinoid-conjugated liposome administration group (Allo+siRNA), both the dermal thickening and the amount of collagen are significantly decreased as compared with the vehicle administration group (Allo+PBS), and from the results shown in FIG. 16, it is found that also the expression of HSP47 is significantly decreased as compared with the vehicle administration group (Allo). Further, from the results of the analysis of the thymus cells or the spleen cells shown in FIGS. 17 to 18, it is found that the treatment with the siRNA-containing retinoid-conjugated liposomes does not significantly affect the number of CD4⁺ and CD8⁺ cells in the thymus (FIG. 17A), the number of T cells in the spleen (FIGS. 17B and C), and the colonization ratio of the donor T cells and hematopoietic cells (FIG. 18).

### Example 6: In Vivo Distribution of siRNA-Containing Retinoid-Conjugated Liposomes

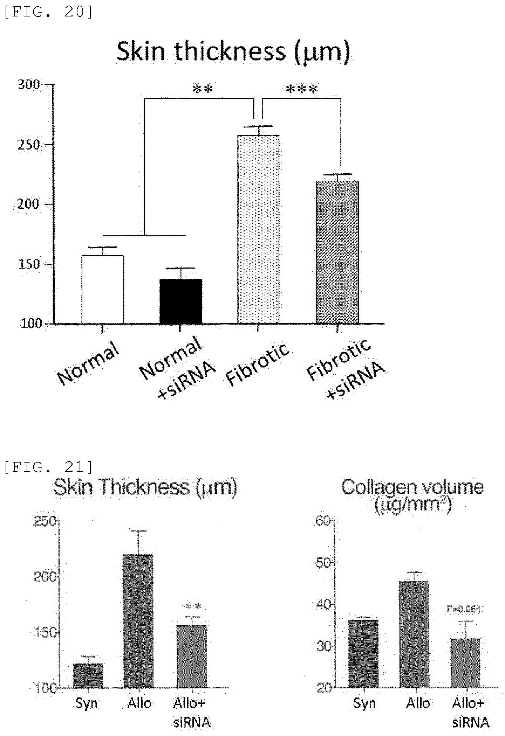

In C57BL/6 mice with skin fibrogenesis induced by administering 100 µg of BLM to a dorsal region every day for 21 days from day 1 to day 21, retinoid-conjugated liposomes labeled with a fluorescent dye Dy647 (including a mixture of siRNA 1 labeled with Dy647 (40%) and unlabeled siRNA 2 (60%)) were intravenously injected 3 times every two hours at a dose of 4.5 mg/kg·BW on day 22. After 2 hours from the final injection, skin samples of a region in which skin fibrogenesis was induced and a normal region were collected and incubated overnight in 4% paraformaldehyde, and thereafter incubated for 24 hours in 30% sucrose. A frozen section was prepared, and stained with DAPI, and then observed using a fluorescence microscope. As shown in FIG. 19, fluorescence by Dy647 was observed in the skin tissue (Fibrotic) in the region where skin fibrogenesis was induced, and it is confirmed that the label was delivered to the lesion site by the liposomes, however, fluorescence by Dy647 was not observed in the skin tissue (Normal) in the normal region. Further, as shown in FIG. 20, the siRNA-containing retinoid-conjugated liposomes significantly reduced the dermal thickness in the fibrogenesis-induced region as compared with the control ("Fibrotic" versus "Fibrotic+siRNA"), but did not significantly affect the dermal thickness in the normal region ("Normal" versus "Normal+siRNA").

### Example 7: Improvement of Established Skin GVHD

A treatment was performed in the same manner as in Example 5 except that the administration of the siRNA-containing retinoid-conjugated liposomes or the vehicle was performed from day 21 to day 41 after skin GVHD was established. From the results shown in FIG. 21, it is found that even the established skin GVHD can be improved by the siRNA-containing retinoid-conjugated liposomes.

As shown by the above-mentioned results, the composition of the present disclosure has suppressed skin fibrogenesis by different mechanisms of bleomycin-induced skin fibrogenesis and skin GVHD, and therefore, it is considered that the composition can be widely used in a treatment of skin fibrogenesis due to various diseases or a traumatic or surgical scar in the skin, a treatment of a keloid, etc.

Further, from the above results, it was revealed that the composition of the present disclosure can exhibit its effect by systemic administration. In cGVHD, etc., fibrogenesis becomes prominent not only in the skin, but also in the organs in the whole body, and in order to deal with fibrogenesis in such a wide range of organs, a drug that can be systematically administered such as the composition of the present disclosure is preferred.

Moreover, from the above results, it was revealed that the composition of the present disclosure does not significantly affect the number or composition of immune cells derived from a donor by allogeneic bone marrow stem cell transplantation. This suggests that the composition of the present disclosure does not significantly affect GVL/GVT, and indicates the usefulness of the composition of the present disclosure for a treatment utilizing GVL/GVT such as a treatment of a tumor by a low-intensity conditioning regimen.

Incidentally, in the experiment using the cGVHD model, diarrhea occurred due to intestinal inflammation by cGVHD, and debilitation or death was observed in mice (FIG. 9). This suggests that the composition of the present disclosure containing siRNA for HSP47 does not have an effect on symptoms that are not associated with such fibrogenesis. However, it is considered that such symptoms other than fibrogenesis can be dealt with by using another therapeutic method in combination . Further, the fact that although the above-mentioned composition was administered to model animals having such a serious and lethal disease, the composition showed no harmful effects as compared with the PBS administration group serving as the control suggests that the composition of the present disclosure has high safety.

## Claims

1. A carrier for delivering a substance to extracellular matrix-producing cells in the skin, comprising a retinoid as a component for promoting the delivery of a substance to extracellular matrix-producing cells in the skin.

2. The carrier according to claim 1, wherein the retinoid is contained as a retinoid conjugate.

3. The carrier according to claim 1 or 2, further comprising a lipid.

4. A pharmaceutical composition for treating skin fibrosis, comprising the carrier according to any one of claims 1 to 3, and a drug that controls the activity or growth of extracellular matrix-producing cells in the skin.

5. A pharmaceutical composition for regenerating a normal skin tissue from a fibrotic skin tissue, comprising the carrier according to any one of claims 1 to 3, and a drug that controls the activity or growth of extracellular matrix-producing cells in the skin.

6. The pharmaceutical composition according to claim 4 or 5, wherein the drug that controls the activity or growth of extracellular matrix-producing cells in the skin is selected from the group consisting of a substance that inhibits the production or secretion of an extracellular matrix component, a cell growth inhibitor, an apoptosis inducer, and a TIMP inhibitor.

7. The pharmaceutical composition according to claim 6, wherein the substance that inhibits the production or secretion of an extracellular matrix component is an HSP47 inhibitor.

8. The pharmaceutical composition according to any one of claims 4 to 7, which is in a form to be prepared before use.

9. A kit for preparing the pharmaceutical composition according to any one of claims 4 to 8, comprising one or more containers containing a drug that controls the activity or growth of extracellular matrix-producing cells in the skin, a retinoid and/or a retinoid conjugate, and a carrier constituent substance other than the retinoid as needed singly or in combination.

10. A method for producing a carrier for delivering a substance to extracellular matrix-producing cells in the skin, comprising a step of blending a retinoid and/or a retinoid conjugate as a component for promoting the delivery of a substance to extracellular matrix-producing cells in the skin.

11. A method for producing a pharmaceutical composition for treating skin fibrosis or a pharmaceutical composition for regenerating a normal skin tissue from a fibrotic skin tissue, comprising a step of blending a retinoid as a component for promoting the delivery of a substance to extracellular matrix-producing cells in the skin and a drug that controls the activity or growth of extracellular matrix-producing cells in the skin as an active ingredient.


SEQUENCE LISTING

<110>  NITTO DENKO CORPORATION

<120>  AGENT FOR TREATING SKIN FIBROSIS

<130>  PCT2839ND

<160>  39

<170>  PatentIn version 3.5

<210>  1
<211>  2208
<212>  RNA
<213>  Homo sapiens

<400>  1
ucuuuggcuu uuuuuggcgg agcuggggcg cccuccggaa gcguuuccaa cuuuccagaa      60

guuucucggg acgggcagga gggggugggg acugccauau auagaucccg ggagcagggg     120

agcgggcuaa gaguagaauc gugucgcggc ucgagagcga gagucacguc ccggcgcuag     180

cccagcccga cccaggccca ccguggugca cgcaaaccac uuccuggcca ugcgcucccu     240

ccugcuucuc agcgccuucu gccuccugga ggcggcccug gccgccgagg ugaagaaacc     300

ugcagccgca gcagcuccug gcacugcgga gaaguugagc cccaaggcgg ccacgcuugc     360

cgagcgcagc gccggccugg ccuucagcuu guaccaggcc auggccaagg accaggcagu     420

ggagaacauc cuggugucac ccgugguggu ggccucgucg cuagggcucg ugucgcuggg     480

cggcaaggcg accacggcgu cgcaggccaa ggcagugcug agcgccgagc agcugcgcga     540

cgaggaggug cacgccggcc ugggcgagcu gcugcgcuca cucagcaacu ccacggcgcg     600

caacgugacc uggaagcugg gcagccgacu guacggaccc agcucaguga gcuucgcuga     660

ugacuucgug cgcagcagca agcagcacua caacugcgag cacuccaaga ucaacuuccg     720

cgacaagcgc agcgcgcugc aguccaucaa cgagugggcc gcgcagacca ccgacggcaa     780

gcugcccgag gucaccaagg acguggagcg cacggacggc gcccugcuag ucaacgccau     840

guucuucaag ccacacuggg augagaaauu ccaccacaag augguggaca accguggcuu     900

cauggugacu cgguccuaua ccgugggugu caugaugaug caccggacag gccucuacaa     960

cuacuacgac gacgagaagg aaaagcugca aaucguggag augccccugg cccacaagcu    1020

cuccagccuc aucauccuca ugccccauca cguggagccu cucgagcgcc uugaaaagcu    1080

gcuaaccaaa gagcagcuga agaucuggau ggggaagaug cagaagaagg cuguugccau    1140

cuccuugccc aagggugugg uggaggugac ccaugaccug cagaaacacc uggcugggcu    1200

gggccugacu gaggccauug acaagaacaa ggccgacuug ucacgcaugu caggcaagaa    1260

ggaccuguac cuggccagcg uguuccacgc caccgccuuu gaguuggaca cagauggcaa    1320

ccccuuugac caggacaucu acgggcgcga ggagcugcgc agccccaagc uguucuacgc    1380

cgaccacccc uucaucuucc uagugcggga cacccaaagc ggcucccugc uauucauugg 1440

gcgccugguc cggccuaagg gugacaagau gcgagacgag uuauagggcc ucagggugca 1500

cacaggaugg caggaggcau ccaaaggcuc cugagacaca ugggugcuau ugggguuggg 1560

ggggagguga gguaccagcc uuggauacuc cauggggugg ggguggaaaa acagaccggg 1620

guucccgugu gccugagcgg accuucccag cuagaauuca cuccacuugg acaugggccc 1680

cagauaccau gaugcugagc ccggaaacuc cacauccugu gggaccuggg ccauagucau 1740

ucugccugcc cugaaagucc cagaucaagc cugccucaau caguauucau auuuauagcc 1800

agguaccuuc ucaccuguga gaccaaauug agcuaggggg gucagccagc ccucuucuga 1860

cacuaaaaca ccucagcugc cuccccagcu cuaucccaac cucucccaac uauaaaacua 1920

ggugcugcag ccccugggac caggcacccc cagaaugacc uggccgcagu gaggcggauu 1980

gagaaggagc ucccaggagg ggcuucuggg cagacucugg ucaagaagca ucgugucugg 2040

cguugugggg augaacuuuu uguuuuguuu cuuccuuuuu uaguucuuca aagauaggga 2100

gggaaggggg aacaugagcc uuuguugcua ucaauccaag aacuuauuug uacauuuuuu 2160

uuuucaauaa aacuuuucca augacauuuu guuggagcgu ggaaaaaa 2208

<210> 2
<211> 19
<212> RNA
<213> Artificial Sequence

<220>
<223> A1-1 sense

<400> 2
gagacacaug ggugcuauu 19

<210> 3
<211> 19
<212> RNA
<213> Artificial Sequence

<220>
<223> A1-1 antisense

<400> 3
aauagcaccc augugucuc 19

<210> 4
<211> 19
<212> RNA
<213> Artificial Sequence

<220>
<223> A1-2 sense

<400> 4

acaagaugcg agacgaguu                                                    19

<210>  5
<211>  19
<212>  RNA
<213>  Artificial Sequence

<220>
<223>  A1-2 antisense

<400>  5
aacucgucuc gcaucuugu                                                    19

<210>  6
<211>  19
<212>  RNA
<213>  Artificial Sequence

<220>
<223>  A1-3 sense

<400>  6
ccuuugacca ggacaucua                                                    19

<210>  7
<211>  19
<212>  RNA
<213>  Artificial Sequence

<220>
<223>  A1-3 antisense

<400>  7
uagauguccu ggucaaagg                                                    19

<210>  8
<211>  19
<212>  RNA
<213>  Artificial Sequence

<220>
<223>  A1-4 sense

<400>  8
cggacaggcc ucuacaacu                                                    19

<210>  9
<211>  19
<212>  RNA
<213>  Artificial Sequence

<220>
<223>  A1-4 antisense

<400>  9
aguuguagag gccuguccg                                                    19

<210>  10
<211>  19
<212>  RNA
<213>  Artificial Sequence

<220>
<223>  A1-5 sense

<400>  10
acuccaagau caacuucca                                                    19


<210>  11
<211>  19
<212>  RNA
<213>  Artificial Sequence

<220>
<223>  A1-5 antisense

<400>  11
uggaaguuga ucuuggagu                                                    19


<210>  12
<211>  19
<212>  RNA
<213>  Artificial Sequence

<220>
<223>  A1-6 sense

<400>  12
gacaagaugc gagacgagu                                                    19


<210>  13
<211>  19
<212>  RNA
<213>  Artificial Sequence

<220>
<223>  A1-6 antisense

<400>  13
acucgucucg caucuuguc                                                    19


<210>  14
<211>  19
<212>  RNA
<213>  Artificial Sequence

<220>
<223>  A1-7 sense

<400>  14
ccugagacac augggugcu                                                    19


<210>  15
<211>  19
<212>  RNA

<213>  Artificial Sequence

<220>
<223>  A1-7 antisense

<400>  15
agcacccaug ugucucagg                                                    19


<210>  16
<211>  19
<212>  RNA
<213>  Artificial Sequence

<220>
<223>  A2-1 sense

<400>  16
gagacacaug ggugcuaua                                                    19


<210>  17
<211>  19
<212>  RNA
<213>  Artificial Sequence

<220>
<223>  A2-1 antisense

<400>  17
uauagcaccc augugucuc                                                    19


<210>  18
<211>  19
<212>  RNA
<213>  Artificial Sequence

<220>
<223>  A2-2 sense

<400>  18
acaagaugcg agacgagua                                                    19


<210>  19
<211>  19
<212>  RNA
<213>  Artificial Sequence

<220>
<223>  A2-2 antisense

<400>  19
uacucgucuc gcaucuugu                                                    19


<210>  20
<211>  19
<212>  RNA
<213>  Artificial Sequence

<220>

<223>  A2-3 sense

<400>  20
cggacaggcc ucuacaaca                                                    19


<210>  21
<211>  19
<212>  RNA
<213>  Artificial Sequence

<220>
<223>  A2-3 antisense

<400>  21
uguuguagag gccuguccg                                                    19


<210>  22
<211>  19
<212>  RNA
<213>  Artificial Sequence

<220>
<223>  A2-4 sense

<400>  22
ugacaagaug cgagacgaa                                                    19


<210>  23
<211>  19
<212>  RNA
<213>  Artificial Sequence

<220>
<223>  A2-4 antisense

<400>  23
uucgucucgc aucuuguca                                                    19


<210>  24
<211>  19
<212>  RNA
<213>  Artificial Sequence

<220>
<223>  A2-5 sense

<400>  24
acuccaagau caacuuccu                                                    19


<210>  25
<211>  19
<212>  RNA
<213>  Artificial Sequence

<220>
<223>  A2-5 antisense

<400>  25

aggaaguuga ucuuggagu                                                    19

<210>  26
<211>  19
<212>  RNA
<213>  Artificial Sequence

<220>
<223>  A2-6 sense

<400>  26
uccugagaca cauggguga                                                    19

<210>  27
<211>  19
<212>  RNA
<213>  Artificial Sequence

<220>
<223>  A2-6 antisense

<400>  27
ucacccaugu gucucagga                                                    19

<210>  28
<211>  19
<212>  RNA
<213>  Artificial Sequence

<220>
<223>  A2-7 sense

<400>  28
gacaagaugc gagacgaga                                                    19

<210>  29
<211>  19
<212>  RNA
<213>  Artificial Sequence

<220>
<223>  A2-7 antisense

<400>  29
ucucgucucg caucuuguc                                                    19

<210>  30
<211>  19
<212>  RNA
<213>  Artificial Sequence

<220>
<223>  A2-8 sense

<400>  30
acaggccucu acaacuaca                                                    19

<210>  31
<211>  19
<212>  RNA
<213>  Artificial Sequence

<220>
<223>  A2-8 antisense

<400>  31
uguaguugua gaggccugu                                                    19


<210>  32
<211>  27
<212>  RNA
<213>  Artificial Sequence

<220>
<223>  Scrambled siRNA sense

<400>  32
cgauucgcua gaccggcuuc auugcag                                           27


<210>  33
<211>  27
<212>  RNA
<213>  Artificial Sequence

<220>
<223>  Scrambled siRNA antisense

<400>  33
gcaaugaagc cggucuagcg aaucgau                                           27


<210>  34
<211>  28
<212>  DNA
<213>  Artificial Sequence

<220>
<223>  serpinh1-P

<400>  34
agccacactg ggatgagaag tttcacca                                          28


<210>  35
<211>  21
<212>  DNA
<213>  Artificial Sequence

<220>
<223>  serpinh1-F

<400>  35
ctgcttgtga acgccatgtt c                                                 21


<210>  36
<211>  20
<212>  DNA

<213>  Artificial Sequence

<220>
<223>  serpinh1-R

<400>  36
tcaccatgaa gccacggttg                                                    20


<210>  37
<211>  22
<212>  DNA
<213>  Artificial Sequence

<220>
<223>  col1a1-P

<400>  37
cggggtcgga gccctcgctt cc                                                 22


<210>  38
<211>  20
<212>  DNA
<213>  Artificial Sequence

<220>
<223>  col1a1-F

<400>  38
accgatggat tcccgttcga                                                    20


<210>  39
<211>  21
<212>  DNA
<213>  Artificial Sequence

<220>
<223>  col1a1-R

<400>  39
cattaggcgc aggaaggtca g                                                  21